(19) Europäisches Patentamt
European Patent Office
Office européen des brevets

(11) EP 3 536 684 A1

(12) EUROPEAN PATENT APPLICATION

(43) Date of publication:
**11.09.2019 Bulletin 2019/37**

(21) Application number: **18160274.9**

(22) Date of filing: **06.03.2018**

(51) Int Cl.:
*C07C 279/14* (2006.01)
*A61P 33/00* (2006.01)
*C07H 21/00* (2006.01)
*C12P 13/02* (2006.01)
*A61P 25/28* (2006.01)
*A61P 35/04* (2006.01)
*A61P 35/00* (2006.01)
*A61K 31/155* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Leibniz-Institut für Naturstoff-Forschung und Infektionsbiologie 07745 Jena (DE)**
- **Friedrich-Schiller-Universität Jena 07743 Jena (DE)**

(72) Inventors:
- **Beemelmanns, Christine 07749 Jena (DE)**
- **Guo, Huijuan 07745 Jena (DE)**
- **Höfgen, Sandra 07747 Jena (DE)**
- **Rischer, Maja 07749 Jena (DE)**
- **Raguz , Luka 07745 Jena (DE)**
- **Keiff, Francois 07743 Jena (DE)**
- **Goris, Tobias 10245 Berlin (DE)**

(74) Representative: **Forstmeyer, Dietmar**
**BOETERS & LIECK**
**Oberanger 32**
**80331 München (DE)**

(54) **BARNESIN A, DERIVATIVES AND USES THEREOF**

(57) This invention relates to a compound according to general formula (1), which acts as a selective cysteine protease inhibitor; to a pharmaceutical composition containing one or more of the compound(s) of the invention; to a combination preparation containing at least one compound of the invention and at least one further active pharmaceutical ingredient; and to uses of said compound(s), including the use as a medicament.

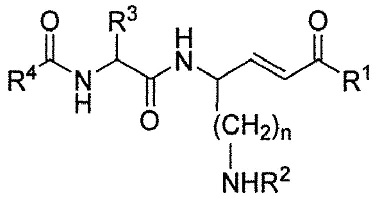

(I)

EP 3 536 684 A1

## Description

### Field of the invention

This invention relates to a compound according to general formula (I), which acts as a selective cysteine protease inhibitor; to a pharmaceutical composition containing one or more of the compound(s) of the invention; to a combination preparation containing at least one compound of the invention and at least one further active pharmaceutical ingredient; and to uses of said compound(s), including the use as a medicament.

### Background of the invention

Natural products of microbial origin have been a rich source of compounds for drug discovery. In particular, actinomycetes and myxobacteria are prime producers of industrially important natural products. However, studies of such well-investigated organisms have led to high rediscovery rate of already known natural product classes. Yet, microbial natural products are still one of the most promising sources for novel drugs. This is, because natural products own an element of structural complexity which allows for the specific and effective inhibition of many targets (e.g. proteins, DNA, RNA). For instance, nonribosomally synthesized peptides and polyketides are classes of secondary metabolites in bacteria, fungi, and plants that exhibit a wide range of bioactivities which are interesting for pharmaceutical applications.

Global genome mining efforts implied the general abundance of secondary metabolite gene clusters within all bacterial phyla (Ziemert and Jensen, Methods in enzymology 2012; 517:161). The increasing number of known genome sequences in combination with bioinformatic tools allows identifying so far uncharacterized and interesting putative biosynthetic gene clusters in microorganisms that may encode novel bioactive secondary metabolites.

Epsilonproteobacteria are among those that have so far been neglected in the characterization of unprecedented secondary metabolites and biosynthetic pathways. Bacteria belonging to the genus *Sulfuruspirillum* (Epsilonproteobacteria) are frequently found in sediments, aquatic habitats (fresh and salt water), oil reservoirs, and sewage plants, and include the non-dehalogenating species *Sulfurospirillum barnesii* (Goris and Dieckert, The genus Sulfurospirillum. In Organohalide-respiring bacteria. Adrian, L., and Löffler, F.E. (eds). Berlin Heidelberg: Springer 2016, Goris et al, Environmental microbiology 2014; 16(11): 3562-3580).

Cysteine proteases are validated targets for treatment of a number of diseases, including neurodegenerative disorders, e.g. Alzheimer's disease, (Siklos et al., Acta Pharmaceutica Sinica B 2015; 5(6): 506-519); parasitic infections, e.g. Chagas disease and human African trypanosomiasis, (Ferreira and Andricopulo, Pharmacology & Therapeutics 2017; 180: 49-61); and invasive and metastatic cancers (Mason and Joyce, Trends in Cell Biology 2011, 21(4): 228-237).

Although much progress has been made in the development of antitumor agents, cancer is still one of the leading causes of death. In addition, many of the current therapies are associated with severe side effects. Similar considerations apply to Chagas disease and human African trypanosomiasis, which are endemic life-threatening conditions in Latin America and Africa for the treatment of which only a few drugs with serious efficacy and safety drawbacks are available (Ferreira and Andricopulo, *loc. cit.).* Available drug therapies for neurodegenerative disorders are also dismal as many suffer from serious efficacy and toxicity problems.

In view of the deficits of the prior art and the severe conditions associated with a pathophysiological level of cysteine proteases, both acute and chronic, there is a need for novel cysteine protease inhibitors.

### Summary and description of the invention

The present invention was made in view of the prior art and the needs described above, and, therefore, the object of the present invention is to provide novel cysteine protease inhibitors according to general formula (I). Other objects of the present invention are to provide a pharmaceutical composition comprising at least one cysteine protease inhibitor as described herein; a combination preparation containing at least one compound of the invention and at least one further active pharmaceutical ingredient; and uses of the compound(s) of the invention, including the use as a medicament.

These objects are solved by the subject-matter of the attached claims as will become apparent upon reference to the following description and definitions. In particular, the inventors established that the compound according to the invention is a potent selective inhibitor of cysteine proteases.

These surprising and unexpected results allow an alternative therapeutic, preventive and/or curative role to be conceived for the compound according to the invention in the prevention and/or treatment of various conditions or disorders associated with a pathophysiological level of a cysteine protease, including neurodegenerative disorders, e.g. Alzheimer's disease; parasitic infections, e.g. Chagas disease and human *African trypanosomiasis;* and invasive and metastatic cancers.

Accordingly, the present invention is directed to a compound of the general formula (I):

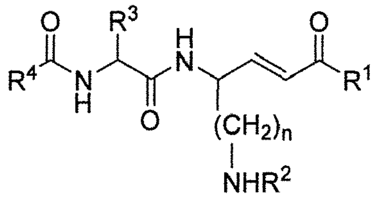

(I)

or a pharmacologically acceptable salt thereof, wherein

$R^1$ represents a hydrogen atom, $-OR^{11}$, $-NR^{11}R^{12}$; or a $(C_1\text{-}C_6)$alkyl, $(C_2\text{-}C_6)$alkenyl, $(C_2\text{-}C_6)$ alkinyl, or $(C_3\text{-}C_6)$ cycloalkyl group, all of which groups may optionally be substituted;
$R^{11}$ and $R^{12}$ each, independently of one another, represents a hydrogen atom, or a $(C_1\text{-}C_6)$alkyl, $(C_2\text{-}C_6)$alkenyl, $(C_2\text{-}C_6)$ alkinyl or $(C_1\text{-}C_6)$heteroalkyl group, all of which groups may optionally be substituted;
$R^2$ is a hydrogen atom, a group of formula $-C(=NH)NH_2$, or a group of formula $-C(=O)R^{21}$;
$R^{21}$ represents a $(C_1\text{-}C_6)$alkyl, $(C_2\text{-}C_6)$alkenyl, $(C_2\text{-}C_6)$ alkinyl or $(C_1\text{-}C_6)$heteroalkyl group; all of which groups may optionally be substituted;
$R^3$ is an amino acid side chain; a hydrogen atom, a halogen atom, OH; or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group; all of which groups may optionally be substituted;
$R^4$ is an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group; all of which groups may optionally be substituted; and
n is an integer of from 1 to 6.

Compounds are usually described herein using standard nomenclature or the definitions presented below. For compounds having asymmetric centers, it should be understood that (unless otherwise specified) all of the optical isomers and mixtures thereof are encompassed. Compounds with two or more asymmetric elements can also be present as mixtures of diastereomers. In addition, compounds with carbon-carbon double bonds may occur in Z- and E- forms, with all isomeric forms of the compounds being included in the present invention unless otherwise specified. Where a compound exists in various tautomeric forms, a recited compound is not limited to any one specific tautomer, but rather is intended to encompass all tautomeric forms. Recited compounds are further intended to encompass compounds in which one or more atoms are replaced with an isotope (*i.e.,* an atom having the same atomic number but a different mass number). By way of general example, and without limitation, isotopes of hydrogen include tritium and deuterium and isotopes of carbon include $^{11}C$, $^{13}C$, and $^{14}C$.

Compounds according to the formulas provided herein, which have one or more stereogenic center(s), have an enantiomeric excess of at least 50%. For example, such compounds may have an enantiomeric excess of at least 60%, 70%, 80%, 85%, 90%, 95%, or 98%. Some embodiments of the compounds have an enantiomeric excess of at least 99%. It will be apparent that single enantiomers (optically active forms) can be obtained by asymmetric synthesis, synthesis from optically pure precursors or by resolution of the racemates. Resolution of the racemates can be accomplished, for example, by conventional methods such as crystallization in the presence of a resolving agent, or chromatography, using, for example a chiral HPLC column.

The compound according to the invention is described herein using a general formula that includes variables such as, *e.g.* R, $R^1$-$R^2$, $R^3$, and $R^4$. Unless otherwise specified, each variable within such a formula is defined independently of any other variable, and any variable that occurs more than one time in a formula is defined independently at each occurrence. Thus, for example, if a group is shown to be substituted with 0-2 R*, the group may be unsubstituted, or substituted with 1 or 2 group(s) R*, wherein R* at each occurrence is selected independently from the corresponding definition of R*. Also, it should be understood that combinations of substituents and/or variables are permissible only if such combinations result in stable compounds, *i.e.,* compounds that can be isolated, characterized and tested for biological activity.

As used herein a wording defining the limits of a range of length such as, e. g., "from 1 to 5" means any integer from 1 to 5, i. e. 1, 2, 3, 4 and 5. In other words, any range defined by two integers explicitly mentioned is meant to comprise and disclose any integer defining said limits and any integer comprised in said range. For example, the term "$C_1$-$C_6$" refers to 1 to 6, *i.e.* 1, 2, 3, 4, 5 or 6, carbon atoms. Further, the prefix "(Cx-y)" as used herein means that the chain, ring or combination of chain and ring structure as a whole, indicated in direct association of the prefix, may consist of a minimum of x and a maximum of y carbon atoms (i.e. $x < y$), wherein x and y represent integers defining the limits of the length of the chain (number of carbon atoms) and/or the size of the ring (number of carbon ring atoms).

A "synthetic compound" or "a not naturally occurring derivative" of a compound disclosed herein is a compound that is chemically distinct from the natural compound, e.g. different stereochemistry, modified by a substituent, etc.

A "pharmacologically acceptable salt" of a compound disclosed herein is an acid or base salt that is generally considered in the art to be suitable for use in contact with the tissues of human beings or animals without excessive toxicity or carcinogenicity, and preferably without irritation, allergic response, or other problem or complication. Such pharmaceutical salts include mineral and organic acid salts of basic residues such as amines, as well as alkali or organic salts of acidic residues such as carboxylic acids.

Suitable pharmaceutical salts include, but are not limited to, salts of acids such as hydrochloric, phosphoric, hydrobromic, malic, glycolic, fumaric, sulfuric, sulfamic, sulfanilic, formic, toluenesulfonic, methanesulfonic, benzene sulfonic, ethane disulfonic, 2-hydroxyethylsulfonic, nitric, benzoic, 2-acetoxybenzoic, citric, tartaric, lactic, stearic, salicylic, glutamic, ascorbic, pamoic, succinic, fumaric, maleic, propionic, hydroxymaleic, hydroiodic, phenylacetic, alkanoic such as acetic, HOOC-$(CH_2)_n$-COOH where n is any integer from 0 to 4 *(i.e.,* 0, 1, 2, 3, or 4) and the like. Similarly, pharmaceutically acceptable cations include, but are not limited to sodium, potassium, calcium, aluminum, lithium and ammonium. Those of ordinary skill in the art will recognize further pharmacologically acceptable salts for the compounds provided herein. In general, a pharmacologically acceptable acid or base salt can be synthesized from a parent compound that contains a basic or acidic moiety by any conventional chemical method. Briefly, such salts can be prepared by reacting the free acid or base forms of these compounds with a stoichiometric amount of the appropriate base or acid in water or in an organic solvent, or in a mixture of the two. Generally, the use of nonaqueous media, such as ether, ethyl acetate, ethanol, isopropanol or acetonitrile, is preferred.

It will be apparent that each compound of formula (I) may, but need not, be present as a hydrate, solvate or non-covalent complex. In addition, the various crystal forms and polymorphs are within the scope of the present invention. The hydratization/hydration may occur during the process of production or as a consequence of the hygroscopic nature of the initially water free compounds. The solvates and/or hydrates may e.g. be present in solid or liquid form.

A "substituent," as used herein, refers to a molecular moiety that is covalently bonded to an atom within a molecule of interest. For example, a substituent on a ring may be a moiety such as a halogen atom, an alkyl, haloalkyl, hydroxy, cyano, or amino group, or any other substituent described herein that is covalently bonded to an atom, preferably a carbon or nitrogen atom, that is a ring member. The term "substituted," as used herein, means that any one or more hydrogen atom(s) on the designated atom or group (e.g. alkyl, alkoxy, alkoxyalkyl, cycloalkyl, heterocycloalkyl, heteroaryl) is replaced with a selection from the indicated substituents, provided that the designated atom's normal valence or the group's number of possible sites for substitution is not exceeded, and that the substitution results in a stable compound, *i.e.* a compound that can be isolated, characterized and tested for biological activity. When a substituent is oxo, *i.e.,* =O, then 2 hydrogens on the atom are replaced. An oxo group that is a substituent of an aromatic carbon atom results in a conversion of -CH- to -C(=O)- and may lead to a loss of aromaticity. For example, a pyridyl group substituted by oxo is a pyridone. The indication mono-, di-, tri or tetrasubstituted denotes groups having one (mono), two (di), three (tri) or four substituents, provided that the substitution does not exceeded the number of possible sites for substitution and results in a stable compound. For example, a monosubstituted imidazolyl group may be an (imidazolidin-2-on)yl group and a disubstituted isoxazolyl group may be a ((3,5-dimethyl)isoxazolyl) group.

As used herein, "comprising", "including", "containing", "characterized by", and grammatical equivalents thereof are inclusive or open-ended terms that do not exclude additional, unrecited elements or method steps. Yet, "comprising", etc. is also to be interpreted as including the more restrictive terms "consisting essentially of' and "consisting of", respectively.

As used herein, "consisting of' excludes any element, step, or ingredient not specified in the claim.

When trade names are used herein, it is intended to independently include the trade name product formulation, the generic drug, and the active pharmaceutical ingredient(s) of the trade name product.

In general, unless defined otherwise, technical and scientific terms used herein have the same meaning as commonly understood by one of ordinary skill in the art to which this invention belongs, and are consistent with general textbooks and dictionaries.

As used herein, the term amino acid encompasses both "proteinogenic" amino acids and "non-conventional" amino acids. "Proteinogenic amino acids" denote α-amino derivatives of aliphatic carboxylic acids that are naturally incorporated into polypeptides, i.e. the twenty two amino acids, which are selected from the group consisting of Glycine, Leucine, Isoleucine, Valine, Alanine, Phenylalanine, Tyrosine, Tryptophan, Aspartic acid, Asparagine, Glutamic acid, Glutamine, Cysteine, Methionine, Arginine, Lysine, Proline, Serine, Threonine, Histidine, Selenocysteine and Pyrrolysine; and all stereomeric isoforms, i.e. D, L-, D- and L-amino acids thereof, are encompasses. The term "non-conventional amino acid" refers to unnatural amino acids or chemical amino acid analogues, e.g. α,α-disubstituted amino acids, N-alkyl amino acids, homo-amino acids, dehydroamino acids, aromatic amino acids (other than phenylalanine, tyrosine and tryptophan), and ortho-, meta- or para-aminobenzoic acid. Non-conventional amino acids also include compounds which have an amine and carboxyl functional group separated in a 1,3 or larger substitution pattern, such as β-alanine, γ-amino butyric acid, Freidinger lactam, the bicyclic dipeptide (BTD), amino-methyl benzoic acid and others well known in the art. Statine-like isosteres, hydroxyethylene isosteres, reduced amide bond isosteres, thioamide isosteres, urea isosteres, carbamate isosteres, thioether isosteres, vinyl isosteres and other amide bond isosteres known to the art may

also be used. The use of analogues or non-conventional amino acids may improve the stability and biological half-life of the added peptide since they are more resistant to breakdown under physiological conditions. The person skilled in the art will be aware of similar types of substitution which may be made. A non limiting list of non-conventional amino acids which may be used as suitable building blocks for a peptide and their standard abbreviations (in brackets) is as follows: α-aminobutyric acid (Abu), L-N-methylalanine (Nmala), α-amino-α-methylbutyrate (Mgabu), L-N-methylarginine (Nmarg), aminocyclopropane (Cpro), L-N-methylasparagine (Nmasn), carboxylate L-N-methylaspartic acid (Nmasp), aniinoisobutyric acid (Aib), L-N-methylcysteine (Nmcys), aminonorbornyl (Norb), L-N-methylglutamine (Nmgln), carboxylate L-N-methylglutamic acid (Nmglu), cyclohexylalanine (Chexa), L-N-methylhistidine (Nmhis), cyclopentylalanine (Cpen), L-N-methylisolleucine (Nmile), L-N-methylleucine (Nmleu), L-N-methyllysine (Nmlys), L-N-methylmethionine (Nmmet), L-N-methylnorleucine (Nmnle), L-N-methylnorvaline (Nmnva), L-N-methylornithine (Nmorn), L-N-methylphenylalanine (Nmphe), L-N-methylproline (Nmpro), L-N-methylserine (Nmser), L-N-methylthreonine (Nmthr), L-N-methyltryptophan (Nmtrp), D-ornithine (Dorn), L-N-methyltyrosine (Nmtyr), L-N-methylvaline (Nmval), L-N-methylethylglycine (Nmetg), L-N-methyl-t-butylglycine (Nmtbug), L-norleucine (Nle), L-norvaline (Nva), α-methyl-aminoisobutyrate (Maib), α-methyl-γ-aminobutyrate (Mgabu), D-α-methylalanine (Dmala), α-methylcyclohexylalanine (Mchexa), D-α-methylarginine (Dmarg), α-methylcylcopentylalanine (Mcpen), D-α-methylasparagine (Dmasn), α-methyl-α-napthylalanine (Manap), D-α-methylaspartate (Dmasp), α-methylpenicillamine (Mpen), D-α-methylcysteine (Dmcys), N-(4-aminobutyl)glycine (Nglu), D-α-methylglutamine (Dmgln), N-(2-aminoethyl)glycine (Naeg), D-α-methylhistidine (Dmhis), N-(3 -aminopropyl)glycine (Norn), D-α-methylisoleucine (Dmile), N-amino-α-methylbutyrate (Nmaabu), D-α-methylleucine (Dmleu), α-napthylalanine (Anap), D-α-methyllysine (Dmlys), N-benzylglycine (Nphe), D-α-methylmethionine (Dmmet), N-(2-carbamylethyl)glycine (Ngln), D-α-methylornithine (Dmorn), N-(carbamylmethyl)glycine (Nasn), D-α-methylphenylalanine (Dmphe), N-(2-carboxyethyl)glycine (Nglu), D-α-methylproline (Dmpro), N-(carboxymethyl)glycine (Nasp), D-α-methylserine (Dmser), N-cyclobutylglycine (Ncbut), D-α-methylthreonine (Dmthr), N-cycloheptylglycine (Nchep), D-α-methyltryptophan (Dmtrp), N-cyclohexylglycine (Nchex), D-α-methyltyrosine (Dmty), N-cyclodecylglycine (Ncdec), D-α-methylvaline (Dmval), N-cylcododecylglycine (Ncdod), D-N-methylalanine (Dnmala), N-cyclooctylglycine (Ncoct), D-N-methylarginine (Dnmarg), N-cyclopropylglycine (Ncpro), D-N-methylasparagine (Dnmasn), N-cycloundecylglycine (Ncund), D-N-methylaspartate (Dnmasp), N-(2,2-diphenylethyl)glycine (Nbhm), D-N-methylcysteine (Dnmcys), N-(3,3-diphenylpropyl)glycine (Nbhe), D-N-methylglutamine (Dnmgln), N-(3 -guanidinopropyl)glycine (Narg), D-N-methylglutamate (Dnmglu), N-(1-hydroxyethyl)glycine (Ntbx), D-N-methylhistidine (Dnmhis), N-(hydroxyethyl))glycine (Nser), D-N-methylisoleucine (Dnmile), N-(imidazolylethyl))glycine (Nhis), D-N-methylleucine (Dnmleu), N-(3 -indolylyethyl)glycine (Nhtrp), D-N-methyllysine (Dnnilys), N-methyl-γ-aminobutyrate (Nmgabu), N-methylcyclohexylalanine (Nmchexa), D-N-methylmethionine (Dnmmet), D-N-methylornithine (Dnmorn), N-methylcyclopentylalanine (Nmcpen), N-methylglycine (Nala), D-N-methylphenylalanine (Dnmphe), N-methylaminoisobutyrate (Nmaib), D-N-methylproline (Dnmpro), N-(1-methylpropyl)glycine (Nile), D-N-methylserine (Dnmser), N-(2-methylpropyl)glycine (Nleu), D-N-methylthreonine (Dnmthr), D-N-methyltryptophan (Dnmtrp), N-(1-methylethyl)glycine (Nval), D-N-methyltyrosine (Dnmtyr), N-methyla-napthylalanine (Nmanap), D-N-methylvaline (Dnmval), N-methylpenicillamine (Nmpen), γ-aminobutyric acid (Gabu), N-(p-hydroxyphenyl)glycine (Nhtyr), L-/-butylglycine (Tbug), N-(thiomethyl)glycine (Ncys), L-ethylglycine (Etg), penicillamine (Pen), L-homophenylalanine (Hphe), L-α-methylalanine (Mala), L-α-methylarginine (Marg), L-α-methylasparagine (Masn), L-α-methylaspartate (Masp), L-α-methyl-t-butylglycine (Mtbug), L-α-methylcysteine (Mcys), L-methylethylglycine (Metg), L-α-methylglutamine (Mgln), L-α-methylglutamate (Mglu), L-α-methylhistidine (Mhis), L-α-methylhomophenylalanine (Mhphe), L-α-methylisoleucine (Mile), N-(2-methylthioethyl)glycine (Nmet), L-α-methylleucine (Mleu), L-α-methyllysine (Mlys), L-α-methylmethionine (Mmet), L-α-methylnorleucine (Mnle), L-α-methylnorvaline (Mnva), L-α-methylornithine (Morn), L-α-methylphenylalanine (Mphe), L-α-methylproline (Mpro), L-α-methylserine (Mser), L-α-methylthreonine (Mthr), L-α-methyltryptophan (Mtrp), L-α-methyltyrosine (Mtyr), L-α-methylvaline (Mval), L-N-methylhomophenylalanine (Nmhphe), N-(N-(2,2-diphenylethyl)carbamylmethyl)glycine (Nnbhm), N-(N-(3 ,3 - diphenylpropyl)carbamylmethyl)glycine (Nnbhe), 1-carboxy-1-(2,2-diphenyl-ethylamino)cyclopropane (Nmbc), L-O-methyl serine (Omser), L-O-methyl homoserine (Omhser).

The expression alkyl or alkyl group denotes a saturated, straight-chain or branched hydrocarbon group that contains from 1 to 20 carbon atoms, preferably from 1 to 12 carbon atoms, more preferably from 1 to 6 carbon atoms, or the number of carbon atoms indicated in the prefix. If an alkyl is substituted, the substitution may take place, independently of one another, by mono-, di-, or tri-substitution of individual carbon atoms of the molecule, e.g. 1, 2, 3, 4, 5, 6, or 7 hydrogen atom(s) may, at each occasion independently, be replaced by a selection from the indicated substituents. The foregoing also applies if the alkyl group forms a part of a group, e.g. haloalkyl, hydroxyalkyl, alkylamino, alkoxy, or alkoxyalkyl. Examples of an alkyl group include methyl, ethyl, propyl, isopropyl, n-butyl, iso-butyl, sec-butyl, *tert-butyl,* n-pentyl, iso-pentyl, n-hexyl, 2,2-dimethylbutyl, or n-octyl, and examples of a substituted alkyl group or a group where the alkyl forms a part of a group, include haloalkyl, e.g. a trifluoromethyl or a difluoromethyl group; hydroxyalkyl, e.g. hydroxymethyl or 2-hydroxyethyl group, and a methoxymethyl group. The term "($C_{1-6}$) alkyl" includes, for example, $H_3C$-, $H_3C-CH_2$-, $H_3C-CH_2-CH_2$-, $H_3C-CH(CH_3)$-, $H_3C-CH_2-CH_2-CH_2$-, $H_3C-CH_2-CH(CH_3)$-, $H_3C-CH(CH_3)-CH_2$, $H_3C-C(CH_3)_2$-, $H_3C-CH_2-CH_2-CH_2-CH_2$-, $H_3C-CH_2-CH_2-CH(CH_3)$-, $H_3C-CH_2-CH(CH_3)-CH_2$-, $H_3C-CH(CH_3)-CH_2-CH_2$-,

$H_3C-CH_2-C(CH_3)_2-$, $H_3C-C(CH_3)_2-CH_2-$, $H_3C-CH(CH_3)-CH(CH_3)-$, $H_3C-CH_2-CH(CH_2CH_3)-$, $-CH_2CH_2CH_2CH_2CH_2CH_3$, $-CH(CH_3)CH_2CH_2CH_2CH_3$, $(H_3CH_2C)CH(CH_2CH_2CH_3)-$, $-C(CH_3)_2(CH_2CH_2CH_3)$, $-CH(CH_3)CH(CH_3)CH_2CH_3$, and $-CH(CH_3)CH_2CH(CH_3)_2$.

The expression alkoxy or alkoxy group refers to an alkyl group singular bonded to oxygen, i.e. -O-alkyl. The term "($C_1$-$C_6$) alkoxy" includes, for example, methoxy, ethoxy, n-propoxy, isopropoxy, n-butoxy, sec-butoxy, isobutoxy, tert-butoxy, n-pentyloxy , tert-amyloxy- or n-hexyloxy, and accordingly ($C_1$-$C_3$)alkoxy includes methoxy, ethoxy, n-propoxy, or isopropoxy.

The expression alkoxyalkyl or alkoxyalkyl group refers to an alkyl group singular bonded to one or more alkoxy group(s), e.g. -alkyl-O-alkyl or -alkyl-O-alkyl-O-alkyl. The term "($C_2$-$C_5$) alkoxyalkyl" includes, for example, methoxymethyl, methoxyethoxymethyl, and 1-ethoxyethyl.

The expression haloalkyl or haloalkyl group refers to an alkyl group in which one, two, three or more hydrogen atoms have been replaced independently of each other by a halogen atom. The term "($C_1$-$C_3$) haloalkyl" includes, for example, fluoromethyl, difluoromethyl, trifluoromethyl, chloromethyl, dichloromethyl, bromomethyl, dibromomethyl, iodomethyl, (1- or 2-)haloethyl (e.g. (1- or 2-)fluoroethyl or (1- or 2-)chloroethyl), (2- or 3-) halopropyl (e.g. (2- or 3-) fluoropropyl or (2- or 3-) chloropropyl).

The expression hydroxyalkyl or hydroxyalkyl group refers to an alkyl group in which one, two, three or more hydrogen atoms have been replaced independently of each other by a hydroxy (OH) group. The term "($C_1$-$C_4$) hydroxyalkyl" includes, for example, hydroxymethyl, hydroxyethyl, hydroxypropyl and hydroxybutyl.

The expression alkenyl or alkenyl group refers to an at least partially unsaturated, straight-chain or branched hydrocarbon group that contains one or more double bond(s) and from 2 to 20 carbon atoms, preferably from 2 to 12 carbon atoms, more preferably from 2 to 6 carbon atoms, wherein said alkenyl group may be optionally substituted. Examples of an unsubstituted alkenyl group include an ethenyl (vinyl), propenyl (allyl), iso-propenyl, butenyl, isoprenyl and hex-2-enyl group. Preferably, an alkenyl group has one or two, especially one, double bond(s).

The expression alkynyl or alkynyl group refers to an at least partially unsaturated, straight-chain or branched hydrocarbon group that contains one or more triple bond(s) and from 2 to 20 carbon atoms, preferably from 2 to 12 carbon atoms, more preferably from 2 to 6, *e.g.* 2, 3 or 4, carbon atoms, wherein said alkynyl group may be optionally substituted. Examples of an unsubstituted alkynyl group include an ethynyl (acetylenyl), propynyl, butynyl or propargyl group. Preferably, an alkynyl group has one or two, especially one, triple bond(s).

As used herein, the expression heteroalkyl or heteroalkyl group refers to an alkyl, alkenyl or alkynyl group (straigt chain or branched) as defined above, in which one or more, preferably 1 to 8, and more preferably 1, 2, 3 or 4, carbon atom(s) has/have been replaced (each independently of the others) by an oxygen, nitrogen, phosphorus, boron, selenium, silicon or sulphur atom, preferably by an oxygen, sulphur or nitrogen atom, or by a SO or $SO_2$ group, wherein said heteroalkyl group may be optionally substituted. As a result, the expression heteroalkyl encompasses groups containing 1 to 19 carbon atoms, preferably from 1 to 11 carbon atoms, more preferably from 1 to 5, *i.e.* 1, 2, 3, 4 or 5, carbon atoms, and accordingly may also be referred to as $C_1$-$C_{19}$, $C_1$-$C_{11}$, and $C_1$-$C_5$ heteroalkyl, respectively. Preferably, a $C_1$-$C_5$ heteroalkyl group contains from 1 to 5, *e.g.* 1, 2, 3 or 4, carbon atoms and 1, 2, 3 or 4, preferably 1, 2 or 3, heteroatoms selected from oxygen, nitrogen and sulphur (especially oxygen and nitrogen). Especially preferably, the expression heteroalkyl refers to an alkyl group as defined above (straight-chain or branched) in which one or more, preferably 1 to 6, especially preferably 1, 2, 3 or 4, carbon atoms have been replaced by an oxygen, sulfur or nitrogen atom. Examples of heteroalkyl groups are alkylamino, dialkylamino, alkylaminoalkyl, dialkylaminoalkyl, acyl, acylalkyl, alkoxycarbonyl, acyloxy, acyloxyalkyl, carboxyalkylamide, alkoxycarbonyloxy, alkylcarbamoyl, alkylamido, alkylcarbamoylalkyl, alkylamidoalkyl, alkylcarbamoyloxyalkyl, alkylureidoalkyl, alkoxy, alkoxyalkyl, or alkylthio group; all of which groups may be optionally substituted.

Examples of heteroalkyl groups include, for example, groups of formulae: $R^a$-O-$Y^a$-, $R^a$-S-$Y^a$-, $R^a$-N($R^b$)-$Y^a$-, $R^a$-CO-$Y^a$-, $R^a$-O-CO-$Y^a$-, $R^a$-CO-O-$Y^a$-, $R^a$-CO-N($R^b$)-$Y^a$-, $R^a$-N($R^b$)-CO-$Y^a$-, $R^a$-O-CO-N($R^b$)-$Y^a$-, $R^a$-N($R^b$)-CO-O-$Y^a$-, $R^a$-N($R^b$)-CO-N($R^c$)-$Y^a$-, $R^a$-O-CO-O-$Y^a$-, $R^a$-N($R^b$)-C(=N$R^d$)-N($R^c$)-$Y^a$-, $R^a$-CS-$Y^a$-, $R^a$-O-CS-$Y^a$-, $R^a$-CS-O-$Y^a$-, $R^a$-CS-N($R^b$)-$Y^a$-, $R^a$-N($R^b$)-CS-$Y^a$-, $R^a$-O-CS-N($R^b$)-$Y^a$-, $R^a$-N($R^b$)-CS-O-$Y^a$-, $R^a$-N($R^b$)-CS-N($R^c$)-$Y^a$-, $R^a$-O-CS-O-$Y^a$-, $R^a$-S-CO-$Y^a$-, $R^a$-CO-S-$Y^a$-, $R^a$-S-CO-N($R^b$)-$Y^a$-, $R^a$-N($R^b$)-CO-S-$Y^a$-, $R^a$-S-CO-O-$Y^a$-, $R^a$-O-CO-S-$Y^a$-, $R^a$-S-CO-S-$Y^a$-, $R^a$-S-CS-$Y^a$-, $R^a$-CS-S-$Y^a$-, $R^a$-S-CS-N($R^b$)-$Y^a$-, $R^a$-N($R^b$)-CS-S-$Y^a$-, $R^a$-S-CS-O-$Y^a$-, $R^a$-O-CS-S-$Y^a$-, wherein $R^a$ being a hydrogen atom, a $C_1$-$C_6$ alkyl, a $C_2$-$C_6$ alkenyl or a $C_2$-$C_6$ alkynyl group; $R^b$ being a hydrogen atom, a $C_1$-$C_6$ alkyl, a $C_2$-$C_6$ alkenyl or a $C_2$-$C_6$ alkynyl group; $R^c$ being a hydrogen atom, a $C_1$-$C_6$ alkyl, a $C_2$-$C_6$ alkenyl or a $C_2$-$C_6$ alkynyl group; $R^d$ being a hydrogen atom, a $C_1$-$C_6$ alkyl, a $C_2$-$C_6$ alkenyl or a $C_2$-$C_6$ alkynyl group and $Y^a$ being a direct bond, a $C_1$-$C_6$ alkylene, a $C_2$-$C_6$ alkenylene or a $C_2$-$C_6$ alkynylene group. Specific examples of heteroalkyl groups, which may optionally be substituted, include acyl, methoxy, trifluoromethoxy, ethoxy, n-propyloxy, isopropyloxy, tert-butyloxy, methoxymethyl, ethoxymethyl, methoxyethyl, methylamino, ethylamino, dimethylamino, diethylamino, isopropylethylamino, methylaminomethyl, ethylaminomethyl, diisopropylaminoethyl, dimethylaminomethyl, dimethylaminoethyl, acetyl, propionyl, butyryloxy, acetyloxy, methoxycarbonyl, ethoxycarbonyl, isobutyrylamino-methyl, N-ethyl-N-methylcarbamoyl, N-methylcarbamoyl, cyano, nitrile, isonitrile, thiocyanate, isocyanate, isothiocyanate and alkylnitrile.

The expression cycloalkyl or cycloalkyl group refers to a saturated or partially unsaturated cyclic group that contains one or more rings (preferably 1 or 2), containing from 3 to 14 ring carbon atoms, preferably from 3 to 10 (more preferably 3, 4, 5, 6 or 7) ring carbon atoms, wherein the cycloalkyl group may be optionally substituted. In an embodiment a partially unsaturated cyclic group has one, two or more double bonds, such as a cycloalkenyl group. Specific examples of an unsubstituted cycloalkyl group are a cyclopropyl, cyclobutyl, cyclopentyl, spiro[4,5]decanyl, norbornyl, cyclohexyl, cyclopentenyl, cyclohexadienyl, decalinyl, bicyclo[4.3.0]nonyl, cyclopentylcyclohexyl, and a cyclohex-2-enyl group.

The expression heterocycloalkyl or heterocycloalkyl group refers to a cycloalkyl group as defined above in which one or more, preferably 1, 2 or 3, ring carbon atoms have been replaced each independently of the others by an oxygen, nitrogen, or sulphur atom (preferably oxygen or nitrogen), or by a SO or SO2 group. A heterocycloalkyl group has preferably 1 or 2 ring(s) containing from 3 to 10 (more preferably 3, 4, 5, 6 or 7, and most preferably 5, 6 or 7) ring atoms. Examples are an aziridinyl, oxiranyl, thiiranyl, oxaziridinyl, dioxiranyl, azetidinyl, oxetanyl, thietanyl, diazetidinyl, dioxetanyl, dithietanyl, pyrrolidinyl, tetrahydrofuranyl, thiolanyl, phospholanyl, silolanyl, azolyl, thiazolyl, isothiazolyl, imidazolidinyl, pyrazolidinyl, oxazolidinyl, isoxazolidinyl, thiazolidinyl, isothiazolidinyl, dioxolanyl, dithiolanyl, piperazinyl, morpholinyl, thiomorpholinyl, trioxanyl, azepanyl, oxepanyl, thiepanyl, homopiperazinyl, or urotropinyl group. Further examples are a 2-pyrazolinyl group, and also a lactam, a lactone and a cyclic imide. The heterocycloalkyl group can be optionally substituted, and may be saturated or mono-, di- or tri-unsaturated. As a result, the expression heterocycloalkyl group also encompasses a group derived from a carbohydrate or saccharide, such as furanoses or pentoses, e.g. arabinose, ribose, xylose, lyxose or desoxyribose, or pyranoses/ hexoses or derivatives thereof, e.g. allose, altrose, glucose, mannose, gulose, idose, galactose, talose, 6-carboxy-D-glucose, 6-carboxy-D-galactose, *N*-acetylchitosamine, glucosamine, *N*-acetylchondrosamin, fucose, rhamnose, or chinovose.

The expression alkylcycloalkyl or alkylcycloalkyl group refers to a group containing both cycloalkyl and also an alkyl, alkenyl or alkynyl group in accordance with the above definitions, for example alkylcycloalkyl, cycloalkylalkyl, alkylcycloalkenyl, alkenylcycloalkyl and alkynylcycloalkyl groups. An alkylcycloalkyl group preferably contains a cycloalkyl group that contains one or two ring systems having from 3 to 10 (preferably 3, 4, 5, 6 or 7) carbon atoms, and one or two alkyl, alkenyl or alkynyl groups having 1 or 2 to 6 carbon atoms, the cyclic groups being optionally substituted.

The expression heteroalkylcycloalkyl or heteroalkylcycloalkyl group refers to alkylcycloalkyl groups as defined above in which one or more (preferably 1, 2 or 3) carbon atoms have been replaced each independently of the others by an oxygen, nitrogen, silicon, selenium, phosphorus or sulphur atom (preferably oxygen, sulphur or nitrogen). A heteroalkylcycloalkyl group preferably contains 1 or 2 ring systems having from 3 to 10 (preferably 3, 4, 5, 6 or 7) ring atoms, and one or two alkyl, alkenyl, alkynyl or heteroalkyl groups having from 1 or 2 to 6 carbon atoms. Examples of such groups are alkyl-heterocycloalkyl, alkylheterocycloalkenyl, alkenylheterocycloalkyl, alkynylheterocycloalkyl, heteroalkylcycloalkyl, heteroalkylheterocycloalkyl and heteroalkylheterocycloalkenyl, the cyclic groups being optionally substituted and saturated or mono-, di- or tri-unsaturated.

The expressions aryl, Ar or aryl group refer to an aromatic group that contains one or more rings containing from 6 to 14 ring carbon atoms ($C_6$-$C_{14}$), preferably from 6 to 10 ($C_6$-$C_{10}$), more preferably 6 ring carbon atoms, wherein the aryl group may be optionally substituted. Examples of an unsubstituted aryl group include a phenyl, naphthyl, biphenyl, or indanyl group.

The expression heteroaryl or heteroaryl group refers to an aromatic group that contains one or more rings containing from 5 to 14 ring atoms, preferably from 5 to 10 (more preferably 5 or 6) ring atoms, and contains one or more (preferably 1, 2, 3 or 4) oxygen, nitrogen, phosphorus or sulphur ring atoms (preferably O, S or N), wherein the heteroaryl group may be optionally substituted. Examples of an unsubstituted heteroaryl group include 2-pyridyl, 2-imidazolyl, 3-phenylpyrrolyl, thiazolyl, oxazolyl, triazolyl, tetrazolyl, isoxazolyl, indazolyl, indolyl, benzimidazolyl, pyridazinyl, quinolinyl, purinyl, carbazolyl, acridinyl, pyrimidyl, 2,3'-bifuryl, 3-pyrazolyl and isoquinolinyl.

The expression aralkyl or aralkyl group refers to a group containing both, aryl and also alkyl, alkenyl, alkynyl and/or cycloalkyl groups in accordance with the above definitions, wherein the aralkyl group may be optionally substituted. As a result, the expression aralkyl group encompasses groups such as, for example, arylalkyl, arylalkenyl, arylalkynyl, arylcycloalkyl, arylcycloalkenyl, alkylaryl-cycloalkyl and alkylarylcycloalkenyl groups, wherein all of said groups may be optionally substituted. Specific examples of an unsubstituted aralkyl group include toluene, xylene, mesitylene, styrene, benzyl chloride, o-fluorotoluene, 1H-indene, tetralin, dihydronaphthalene, indanone, phenylcyclopentyl, cumene, cyclohexylphenyl, fluorene and indan. An aralkyl group preferably contains one or two aromatic ring systems (1 or 2 rings) containing from 6 to 10 carbon atoms and one or two alkyl, alkenyl and/or alkynyl groups containing from 1 or 2 to 6 carbon atoms and/or a cycloalkyl group containing 5 or 6 ring carbon atoms.

The expression heteroaralkyl or heteroaralkyl group refers to an aralkyl group as defined above in which one or more (preferably 1, 2, 3 or 4) carbon atoms have been replaced each independently of the others by an oxygen, nitrogen, silicon, selenium, phosphorus, boron or sulphur atom (preferably oxygen, sulphur or nitrogen), that is to say to groups containing both aryl or heteroaryl and also alkyl, alkenyl, alkynyl and/or heteroalkyl and/or cycloalkyl and/or heterocycloalkyl groups in accordance with the above definitions, wherein the heteroaralkyl group may be optionally substituted. A heteroaralkyl group preferably contains one or two aromatic ring systems (1 or 2 rings) containing from

5 or 6 to 10 ring carbon atoms and one or two alkyl, alkenyl and/or alkynyl groups containing 1 or 2 to 6 carbon atoms and/or a cycloalkyl group containing 5 or 6 ring carbon atoms, 1, 2, 3 or 4 of those carbon atoms having been replaced each independently of the others by oxygen, sulphur or nitrogen atoms. Examples of heteroaralkyl groups are arylheteroalkyl, arylheterocycloalkyl, arylheterocycloalkenyl, arylalkylheterocycloalkyl, arylalkenylheterocycloalkyl, arylalkynylheterocycloalkyl, arylalkylheterocycloalkenyl, heteroarylalkyl, heteroarylalkenyl, heteroarylalkynyl, heteroarylheteroalkyl, heteroarylcycloalkyl, heteroarylcycloalkenyl, heteroarylheterocycloalkyl, heteroarylheterocycloalkenyl, heteroarylalkylcycloalkyl, heteroarylalkylheterocycloalkenyl, heteroarylheteroalkylcycloalkyl, heteroarylheteroalkylcycloalkenyl, heteroalkylheteroarylalkyl and heteroarylheteroalkylheterocycloalkyl groups; all of which groups may be optionally substituted and the cyclic moieties of said groups being saturated or mono-, di- or tri-unsaturated. Specific examples of a heteroaralkyl group include a tetrahydroisoquinolinyl, benzoyl, 2- or 3-ethylindolyl, 4-methylpyridino, 2-, 3- or 4-methoxyphenyl, 4-ethoxyphenyl, 2-, 3- or 4-carboxyphenylalkyl group.

The expression halogen or halogen atom as preferably used herein means fluorine, chlorine, bromine, or iodine.

The expression "optionally substituted" refers to groups in which one or more hydrogen atoms, e.g. 1 to 7 hydrogen atoms, preferably 1 to 5 hydrogen atoms, and more preferably 1 to 3 hydrogen atoms, have been replaced each independently of the others by fluorine, or chlorine atoms or by OH, =O, SH, =S, $NH_2$, =NH, CN or $NO_2$ groups. The expression "optionally substituted" refers furthermore to groups in which one or more hydrogen atoms, e.g. 1 to 7 hydrogen atoms, preferably 1 to 5 hydrogen atoms, and more preferably 1 to 3 hydrogen atoms, have been replaced each independently of the others by unsubstituted $C_1$-$C_6$alkyl, ($C_1$-$C_6$) haloalkyl (e.g. a fluoromethyl, trifluoromethyl, chloromethyl, (1- or 2-)haloethyl (e.g. (1- or 2-) chloroethyl), or (2- or 3-) halopropyl (e.g. (2- or 3-) fluoropropyl) group), ($C_1$-$C_6$) hydroxyalkyl (e.g. a hydroxymethyl, (1- or 2-)hydroxyethyl, or (2- or 3-) hydroxypropyl group), unsubstituted $C_2$-$C_6$alkenyl, unsubstituted $C_2$-$C_6$alkynyl, unsubstituted $C_1$-$C_6$heteroalkyl (e.g. $C_1$-$C_6$alkoxy; or $C_1$-$C_6$alkoxyalkyl), unsubstituted $C_3$-$C_7$cycloalkyl, unsubstituted $C_2$-$C_7$heterocycloalkyl, unsubstituted $C_6$-$C_{10}$aryl, unsubstituted $C_1$-$C_8$heteroaryl, unsubstituted $C_7$-$C_{12}$aralkyl or unsubstituted $C_2$-$C_{11}$heteroaralkyl groups.

Generally preferred substituents include: halogen atoms (e.g. F, Cl, Br), groups of formula -OH, - O-$C_{1-6}$ alkyl (e.g. -OMe, -OEt, -O-nPr, -O-iPr, -O-nBu, -O-iBu or -O-tBu), -$NH_2$, -$NHC_{1-6}$ alkyl, -N($C_{1-6}$ alkyl)$_2$, -COOH, -$SO_3H$, =O, -$SO_2NH_2$, -$CONH_2$, -CN, -$C_{1-6}$ alkyl (e.g. -Me, -Et, -nPr, -iPr, -nBu, -iBu, - tBu), -$CF_3$, -SH, -S-$C_{1-6}$ alkyl, NHAc, -$NO_2$, -$NHCONH_2$, -$SO_2Me$, and cyclopropyl.

The activity and more specifically the bioactivity of the compounds according to the present invention can be assessed using appropriate assays known to those skilled in the art, e.g. *in vitro* or *in vivo* assays. For instance, the protease inhibition activity against cysteine proteases may be determined according to the procedure of García-Carreño, Biotechnology Education 1992, 3: 145-150 or Hiwasa et al., Cancer letters 1993, 69: 161-165, as utilised in the below, which are thus embodiments of standard *in vitro* assays.

Preferred is a compound of formula (I), or a pharmacologically acceptable salt thereof, wherein the compound, or salt thereof, is a synthetic compound or a not naturally occurring derivative.

Further preferred is a compound of formula (I), or a pharmacologically acceptable salt thereof, wherein $R^1$ is a hydrogen atom, -OH or a $C_{1-3}$ alkoxy group, preferably a methoxy (-$OCH_3$) or ethoxy (-$OC_2H_5$) group; $R^1$ may also represent a C3- or $C_4$ cycloalkyl group, which may be optionally substituted; -$NH_2$, -$NHCH_3$ or $N(CH_3)_2$.

In the compound of formula (I), or a pharmacologically acceptable salt thereof, $R^2$ can be a hydrogen atom, a group of formula -C(=NH)$NH_2$, a group of formula -C(=O)$CH_3$, or a group of formula -C(=O)$CH_2CH_2CH_3$.

In the compound of formula (I), or a pharmacologically acceptable salt thereof, n can preferably be 3 or 4.

In the compound of formula (I), or a pharmacologically acceptable salt thereof, $R^3$ can represent an amino acid side chain of a proteinogenic amino acid, including, for example, a hydrogen atom (glycine), a phenyl group (phenylalanine) or a 4-hydroxyphenyl group (tyrosine); or a group of formula (II):

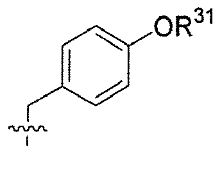

(II),

wherein $R^{31}$ represents a ($C_1$-$C_6$)alkyl, ($C_2$-$C_6$)alkenyl, or ($C_2$-$C_6$) alkinyl group, all of which groups may optionally be substituted. Preferably, $R^{31}$ represents an optionally substituted ($C_1$-$C_4$)alkyl, ($C_2$-$C_4$)alkenyl, or ($C_2$-$C_4$) alkinyl group; and more preferably $R^{31}$ is selected from methyl, prop-2-enyl, prop-2-inyl, but-3-enyl and but-3-inyl.

In the compound of formula (I), or a pharmacologically acceptable salt thereof, $R^4$ can be a $C_{1-7}$ alkyl or $C_{2-7}$ alkenyl group; which groups may optionally be substituted. Preferably, $R^4$ is selected from methyl, heptyl, prop-1-enyl and hept-1-enyl.

The compound of formula (I), or a pharmacologically acceptable salt thereof, includes the compounds depicted

below:

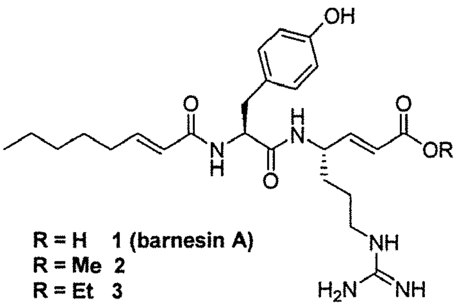

R = H 1 (barnesin A)
R = Me 2
R = Et 3

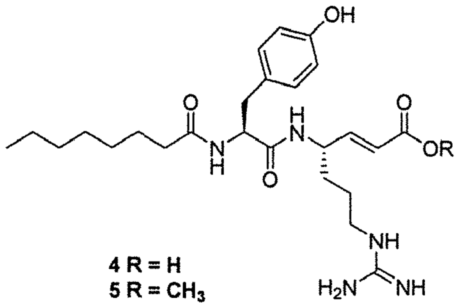

4 R = H
5 R = $CH_3$

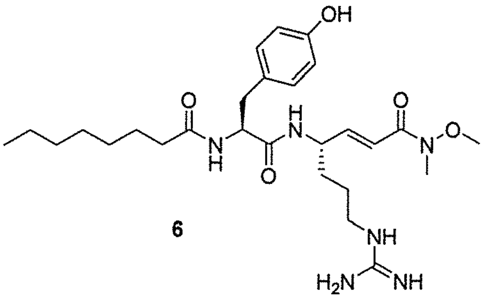

6

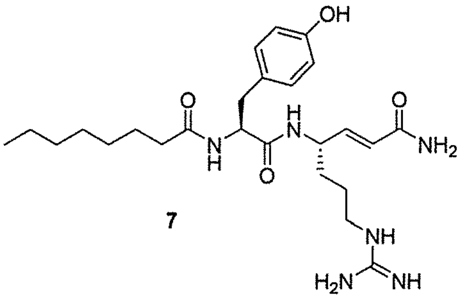

7

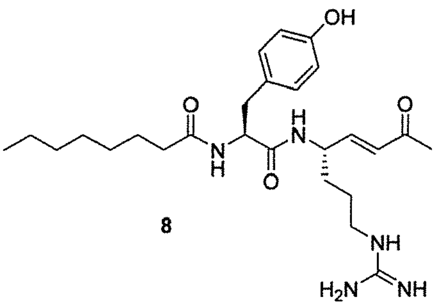

8

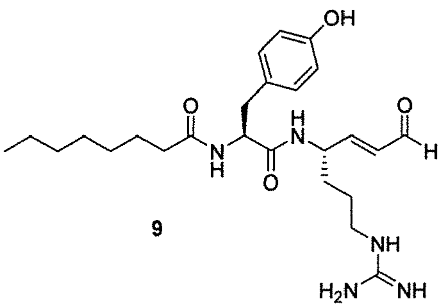

9

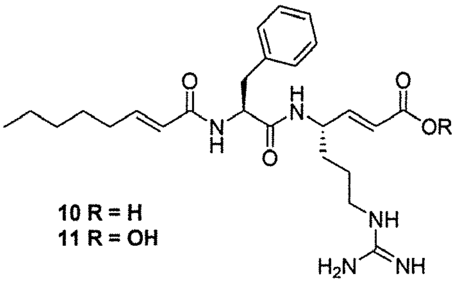

10 R = H
11 R = OH

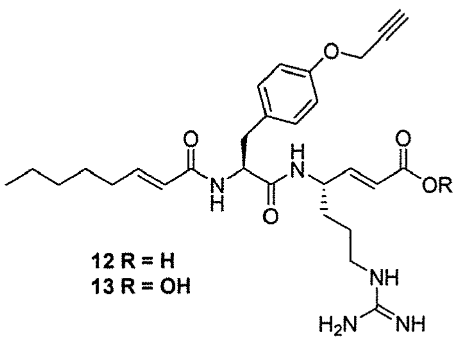

12 R = H
13 R = OH

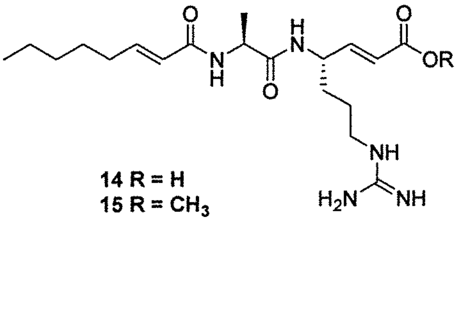

14 R = H
15 R = $CH_3$

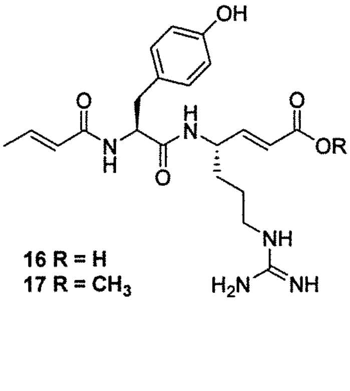

16 R = H
17 R = $CH_3$

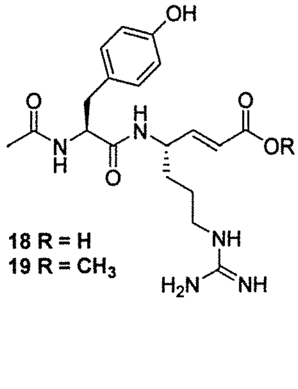

18 R = H
19 R = $CH_3$

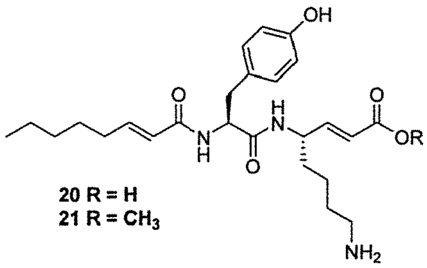

20 R = H
21 R = $CH_3$

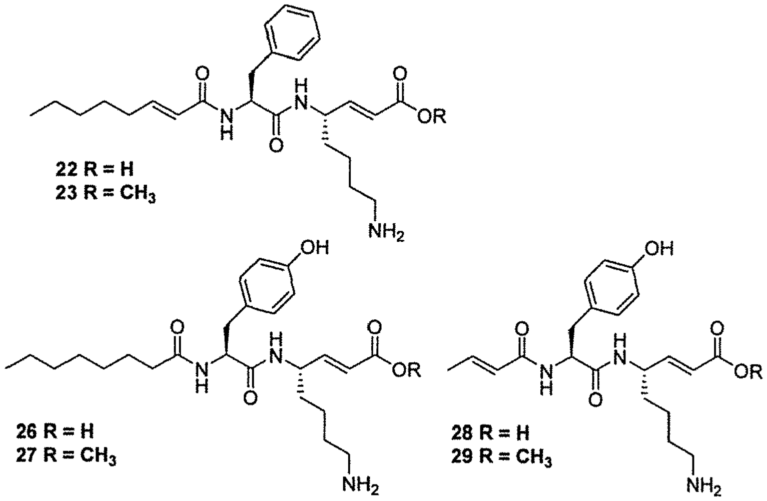

22 R = H
23 R = $CH_3$

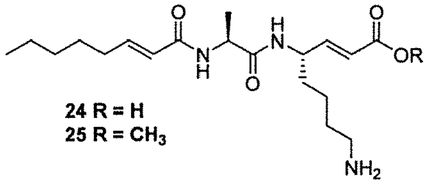

24 R = H
25 R = $CH_3$

26 R = H
27 R = $CH_3$

28 R = H
29 R = $CH_3$

The therapeutic use of a compound of formula (I), of a pharmacologically acceptable salt, solvate or hydrate thereof, and also of a formulation and/or pharmaceutical composition containing the same is within the scope of the present invention. The present invention also relates to the use of a compound of formula (I) as active ingredient in the preparation or manufacture of a medicament.

A pharmaceutical composition according to the present invention comprises at least one compound of formula (I) and, optionally, one or more carrier substance(s), excipient(s) and/or adjuvant(s). Pharmaceutical compositions may additionally comprise, for example, one or more of water, buffers (*e.g.*, neutral buffered saline or phosphate buffered saline), ethanol, mineral oil, vegetable oil, dimethylsulfoxide, carbohydrates (*e.g.*, glucose, mannose, sucrose or dextrans), mannitol, proteins, adjuvants, polypeptides or amino acids such as glycine, antioxidants, chelating agents such as EDTA or glutathione and/or preservatives. Furthermore, one or more other active ingredients may (but need not) be included in the pharmaceutical compositions provided herein. For instance, the compounds of the invention may advantageously be employed in combination with another different antibiotic, an antifungal agent, an anti-viral agent, an antihistamine, a non-steroidal anti-inflammatory drug, a disease modifying anti-rheumatic drug, a cytostatic drug, a drug with smooth muscle activity modulatory activity; or mixtures of the aforementioned.

A pharmaceutical composition may be formulated for any appropriate route of administration, including, for example, parenteral administration. The term parenteral as used herein includes subcutaneous, intradermal, intravascular such as, *e.g.*, intravenous, intramuscular, spinal, intracranial, intrathecal, intraocular, periocular, intraorbital, intrasynovial and intraperitoneal injection, as well as any similar injection or infusion technique.

Carrier substances are, for example, cyclodextrins such as hydroxypropyl β-cyclodextrin, micelles or liposomes, excipients and/or adjuvants. Customary excipients include, for example, inert diluents such as, *e.g.*, calcium carbonate, sodium carbonate, lactose, calcium phosphate or sodium phosphate, granulating and disintegrating agents such as, *e.g.*, corn starch or alginic acid, binding agents such as, *e.g.,* starch, gelatin or acacia, and lubricating agents such as, *e.g.,* magnesium stearate or stearic acid. Examples of adjuvants are aluminum hydroxide, aluminum phosphate, calcium phosphate hydroxide, paraffin oil, squalene, thimerosal, detergents, Freund's complete adjuvant, or Freund's incomplete adjuvant.

For the prevention and/or treatment of conditions or disorders associated with a pathophysiological level of a cysteine protease, including neurodegenerative disorders, e.g. Alzheimer's disease; parasitic infections, e.g. Chagas disease and human African trypanosomiasis; and invasive and metastatic cancers, the dose of the biologically active compound according to the invention may vary within wide limits and may be adjusted to individual requirements. Active compounds according to the present invention are generally administered in a therapeutically effective amount. The expression "therapeutically effective amount" denotes a quantity of the compound(s) that produces a result that in and of itself helps to ameliorate, heal, or cure the respective condition or disease. Preferred doses range from about 0.1 mg to about 140 mg per kilogram of body weight per day (about 0.5 mg to about 7 g per patient per day). The daily dose may be administered as a single dose or in a plurality of doses. The amount of active ingredient that may be combined with the carrier materials to produce a single dosage form will vary depending upon the host treated and the particular mode of administration. Dosage unit forms will generally contain between from about 1 mg to about 500 mg of an active ingredient.

It will be understood, however, that the specific dose level for any particular patient will depend upon a variety of factors including the activity of the specific compound employed, the age, body weight, general health, sex, diet, time of administration, route of administration, and rate of excretion, drug combination (*i.e.* other drugs being used to treat

the patient) and the severity of the particular disease undergoing therapy.

The invention further relates to a combination preparation containing at least one compound according to the invention and at least one further active pharmaceutical ingredient. The combination preparation of the invention can be used as a medicament, in particular in the treatment or prophylaxis of conditions or disorders associated with a pathophysiological level of a cysteine protease, including neurodegenerative disorders, e.g. Alzheimer's disease; parasitic infections, e.g. Chagas disease and human African trypanosomiasis; and invasive and metastatic cancers. Preferably, in the combination preparation of the invention the further active pharmaceutical ingredient can be selected from an antibiotic, an anticancer drug, dopaminergic substances, cholinesterase inhibitors, antipsychotic drugs, analgesic drugs for pain, anti-inflammatories for infections, non-steroidal anti-inflammatory drugs for Alzheimer's disease, oxaboroles, fexinidazole, suramin, pentamidine, benznidazole, and nifurtimox.

Preferred compounds of the invention will have certain pharmacological properties. Such properties include, but are not limited to, bioavailability (especially with regard to oral administration), metabolic stability and sufficient solubility, such that the dosage forms can provide therapeutically effective levels of the compound *in vivo.*

The compound according to the invention, or a pharmacologically acceptable salt thereof, as well as the pharmaceutical composition or combination preparation according to the invention, can be used as a medicament, which can be administered to a patient, *e.g*. parenterally to a human or an other mammal, with dosages as described herein, and will be present within at least one body fluid or tissue of the patient. As used herein, the term "treatment" encompasses both disease-modifying treatment and symptomatic treatment, either of which may be prophylactic, *i.e.,* before the onset of symptoms, in order to prevent, delay or reduce the severity of symptoms, or therapeutic, *i.e.,* after the onset of symptoms, in order to reduce the severity and/or duration of symptoms. In particular, the conditions or diseases that can be ameliorated, prevented or treated using a compound of formula (I), a pharmaceutical composition or a combination preparation according to the invention include conditions or disorders associated with a pathophysiological level of a cysteine protease, including neurodegenerative disorders, e.g. Alzheimer's disease; parasitic infections, e.g. Chagas disease and human *African trypanosomiasis;* and invasive and metastatic cancers. Thus, the present invention also provides methods for treating patients suffering from said diseases. A method for the treatment of a subject which is in need of such treatment comprises the administration of a compound, a pharmaceutical composition, or a combination preparation according to the invention. The term "subject" refers to patients, including, but not limited to primates (especially humans), domesticated companion animals (such as dogs, cats, horses) and livestock (such as cattle, pigs, sheep).

The compound according to the invention, or a pharmacologically acceptable salt thereof, can also have utility as an inhibitor of a proteasome or a cysteine protease, e.g. in *in vivo* or *in vitro* assays. Preferably, the cysteine protease is a cathepsin, including cathepsin B, C, F, H, K, L, O, S, V, X and W, and isoforms thereof; a calpain, including calpain 1 to calpain 15, and isoforms thereof; papain, ficin, a falcipain, including falcipain 1 to falcipain 4, and isoforms thereof; rhodesain (cathepsin L-like protease), and cruzain.

The present invention also provides a synthetic (not naturally occurring) nucleic acid sequence encoding a nonribosomal peptide-synthetase (NRPS) - polyketide synthase (PKS) biosynthetic hybrid cluster capable of synthesizing barnesin A (1), wherein the sequence has a sequence identity to the full-length sequence of SEQ ID NO. 1 from at least 90%, 95%, 96%, 97%, 98%, 98.5%, 99%, or 99.5% to 100%.

The synthetic nucleic acid sequence is a variant derived from the native NRPS - PKS hybrid cluster of *Sulfurospirillum barnesii* which includes cDNAs and may further comprise regulatory sequences, such as promoter and translation initiation and termination sequences, and can further include sequences that facilitate stable maintenance in a host cell, i.e., sequences that provide the function of an origin of replication or facilitate integration into host cell chromosomal or other DNA by homologous recombination. The term "variant" as used herein denotes a polynucleotide that has been modified at one or more positions compared to the native NRPS - PKS hybrid cluster of *Sulfurospirillum barnesii.* Nucleic acids can be, relative to the native NRPS - PKS hybrid cluster, substituted (different), inserted, or deleted, but the variant has generally similar (enzymatic) activity or function as compared to the native NRPS - PKS hybrid cluster.

The term "identity" refers to a property of sequences that measures their similarity or relationship. Identity is measured by dividing the number of identical residues by the total number of residues and multiplying the product by 100.

The term "synthetic" as used herein means that the material, e.g. a nucleic acid sequence, has been synthesized in vitro by well-known chemical synthesis/recombination techniques, and further that the nucleic acid sequence is flanked to a "backbone" nucleic acid to which it is not adjacent in its natural environment. Backbone molecules according to the invention include nucleic acids such as cloning and expression vectors, self-replicating nucleic acids, viruses, integrating nucleic acids and other vectors or nucleic acids used to maintain or manipulate a nucleic acid insert of interest. Recombinant polypeptides of the invention, generated from these nucleic acids can be individually isolated or cloned and tested for a desired activity. Any recombinant expression system can be used, including bacterial, mammalian, yeast, insect or plant cell expression systems.

Also the following methods for producing a compound of formula (I) lie within the scope of the present invention:

chemical synthesis, semisynthesis, and biosynthesis including recombinant techniques.

For instance, a compound of general formula (I) can be produced by a method comprising the steps:

(a) fermenting *Sulfurospirillum barnesii* (DSM 10660); and
(b) separating and retaining the compound according to general formula (I) from the culture broth.

It is understood that the production of compounds of formula (I) is not limited to the use of the particular organism described herein, which is given for illustrative purpose only. The invention also includes the use of any mutants which are capable of producing a compound of formula (I) including natural mutants as well as artificial mutants, e.g. genetically manipulated mutants and the expression of the gene cluster responsible for biosynthesis in a producer strain or by heterologous expression in host strains.

The culturing step can be performed in liquid culture, by growing the respective host cell in media containing one or several different carbon sources, and one or different nitrogen sources. Also salts are essential for growth and production. Suitable carbon sources are different mono-, di-, and polysaccharides like maltose, glucose or carbon from amino acids like peptones. Nitrogen sources are ammonium, nitrate, urea, chitin or nitrogen from amino acids. The following inorganic ions support the growth or are essential in synthetic media: Mg-ions, Ca-ions, Fe-ions, Mn-ions, Zn-ions, K-ions, sulfate-ions, Cl-ions, phosphate-ions. The host cell may be a microorganism, e.g. *Sulfurospirillum barnesii* strain SES-3 (DSM 10660, Genbank accession CP003333.1). Temperatures for growth and production are between 15°C to 40 °C, preferred temperatures are between 25 °C and 35 °C, especially at 28 °C. The pH of the culture solution is from 5 to 8, preferably a pH of 7.2 to 7.4.

A compound of general formula (I) can also be obtained by chemical synthesis in a number of ways well known to one skilled in the art of organic synthesis using usual chemical reaction and synthesis methods. For example, the compounds of the present invention can be synthesized according to Reaction Schemes 1 and 2 shown below using synthetic methods known in the art of synthetic organic chemistry, or variations thereon as appreciated by those skilled in the art, e.g. March's Advanced Organic Chemistry: Reactions, Mechanisms, and Structure, Michael B. Smith, Jerry March, John Wiley & Sons, 2007. Unless indicated otherwise, all variables have the above defined meaning. As starting materials reagents of standard commercial grade can be used without further purification, or can be readily prepared from such materials by routine methods. Those skilled in the art of organic synthesis will recognize that starting materials and reaction conditions may be varied including additional steps employed to produce compounds encompassed by the present invention.

***Reaction Scheme 1:Solution-based synthesis starting from N-protected amino acids.***

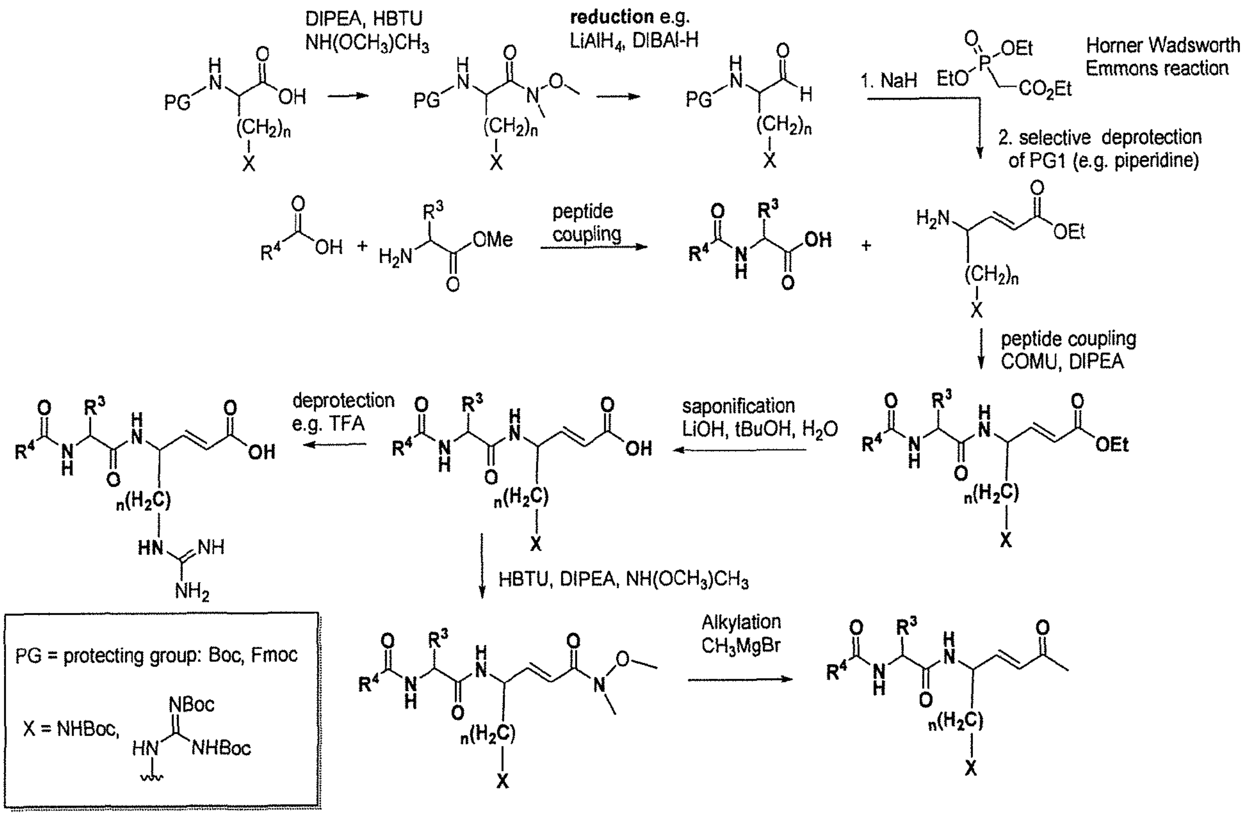


***Reaction Scheme 2: Solid-phase based synthesis starting from resin-bound modified amino acids.***

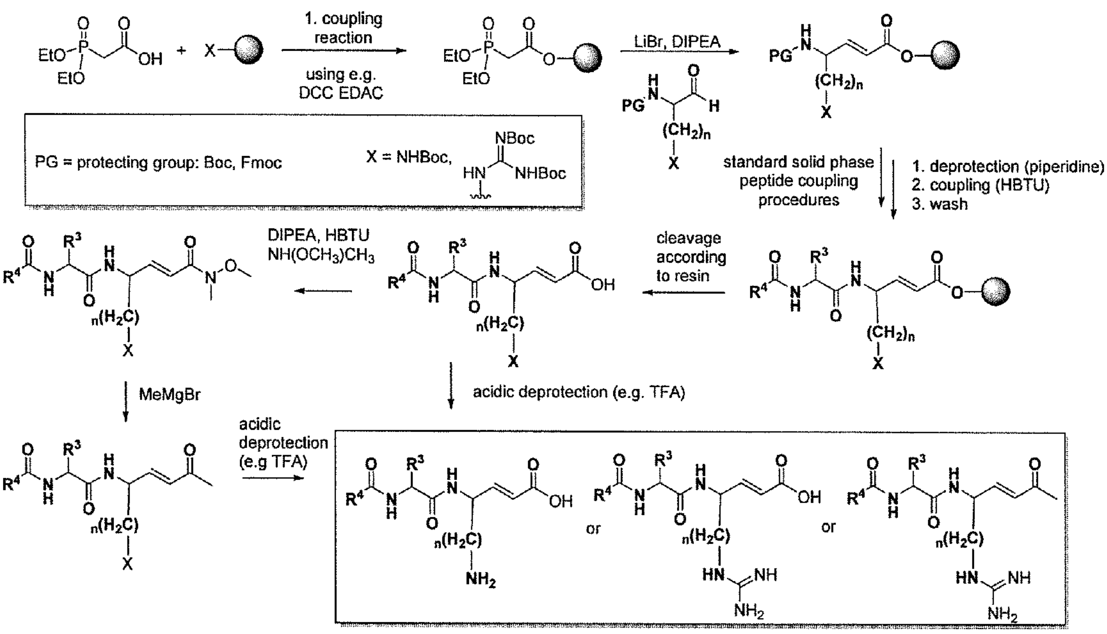


The present invention is now further illustrated by the following examples from which further features, embodiments and advantages of the present invention may be taken.

## EXAMPLES

### General Experimental Procedures

**NMR measurements** were performed on a Bruker AVANCE II 300 MHz, Bruker AVANCE III 500 MHz and a Bruker AVANCE III 600 MHz spectrometer, equipped with a Bruker Cryoplatform. The chemical shifts are reported in parts per million (ppm) relative to the solvent residual peak of DMSO-$d_6$ ($^1$H: 2.50 ppm, quintet; $^{13}$C: 39.52 ppm, heptet), $CDCl_3$ ($^1$H: 7.26 ppm, singlet; $^{13}$C: 77.16 ppm, triplet) and MeOD-$d_4$ ($^1$H: 3.31 ppm, quintet; $^{13}$C: 49.00 ppm, heptet). **LC-ESI-HRMS** measurements were carried out on an Accela UPLC system (Thermo Scientific) coupled with a Accucore C18 column (100 x 2.1 mm, particle size 2.6 μm) combined with a Q-Exactive mass spectrometer (Thermo Scientific) equipped with an elecrospray ion (ESI) source. **UHPLC-MS measurements** were performed on a Shimadzu LCMS-2020 system equipped with single quadrupole mass spectrometer using a Phenomenex Kinetex C18 column (50 x 2.1 mm, particle size 1.7 μm, pore diameter 100 Å). Column oven was set to 40 °C; scan range of MS was set to *m/z* 150 to 2,000 with a scan speed of 10,000 u/s and event time of 0.25 s under positive and negative mode. DL temperature was set to 250 °C with an interface temperature of 350 °C and a heat block of 400 °C. The nebulizing gas flow was set to 1.5 L/min and dry gas flow to 15 L/min. **Semi-preparative HPLC** was performed on a Shimadzu HPLC system using a Phenomenex Luna C18(2) and phenyl-hexyl 250 x 10 mm column (particle size 5 μm, pore diameter 100 Å), or a Biotage Isolera Prime. **IR spectra** were recorded on an FT/IR-4100 ATR spectrometer (JASCO). **Optical rotations** were recorded in MeOH on a P-1020 polarimeter (JASCO). **Solid phase extraction** was carried out using Chromabond C18ec cartridges filled with 1 g and 10 g of octadecyl-modified silica gel (Macherey-Nagel, Germany). **PCR** was performed on a Peqstar 2x Gradient cycler.

**Chemicals:** Methanol (VWR, Germany); water for analytical and preparative HPLC (Millipore, Germany), formic acid (Carl Roth, Germany); acetonitrile (VWR as LC-MS grade), media ingredients (Carl Roth, Germany). All reagents and solvents for synthesis were purchased from Acros Organics, Alfa Aesar, Carbolution Chemicals, Carl Roth, Sigma Aldrich, TCI, Th. Geyer and VWR and used without further purification.

### Example 1 Biosynthesis and biophysical analysis of a compound according to general formula (I) of the present invention - barnesin A (1)

**Cultivation of *Sulfurospirillum barnesii* strain SES-3 (DSM 10660):** Sulfurospirillum spp. were generally cultivated at 28 °C using the following conditions: For solid phase cultivation, S. barnesii was grown microoxically on R2A agar plates incubated in an anaerobic jar with approximately 0.2% oxygen in the gas phase for at least one week. When grown anaerobically, a defined mineral growth medium as described for *S. multivorans* was used with the following modifications: vitamin B12 (cyanocobalamin) and resazurine were omitted and pyruvate (40 mM) was used as electron donor and fumarate (40 mM) as electron acceptor. Small scale cultivations (100 mL) were performed in 200 mL rubber-stoppered serum bottles, large-scale cultivations in 2 L rubber-stoppered Schott bottles containing 1 L medium. Glass bottles used for cultivation were capped with Teflon-coated butyl rubber septa. Growth was monitored photometrically by measuring the optical density at 578 nm. Microaerobic cultivation with the aforementioned medium was performed using 2 L Schott bottles with an initial addition of 2% sterile air into the gas phase into the medium without fumarate as electron acceptor. Inoculation of the medium was performed with 10% of a preculture cultivated until the exponential phase.

**Isolation and characterization:** Cultures were centrifuged at 4000 rpm for 20 min and RT. The cell pellet was harvested and extracted using MeOH (4 °C, overnight), cell debris was filtered off and methanolic extracts added to metabolites at a later step. The culture supernatant was mixed with activated HP20/XAD4 (1:1) resin and stirring overnight (4 °C, 300 rpm). Then, the resin was filtered off, washed with 20% MeOH (if not mentioned otherwise, mixtures refers to MeOH in $ddH_2O$) and metabolites eluted with 50% MeOH and 100% MeOH. Then methanolic cell pellet extracts were added to the 100% MeOH resin eluate. Combined extracts were concentrated under reduced pressure and redissolved in 10% MeOH. The crude extract was loaded on an activated and equilibrated SPE-C18 cartridge and fractionated using 50% and 100% MeOH. The concentrated 100% MeOH fraction was purified first by a semi-preparative HPLC over Luna C18(2) column to obtain subfractions (Fr. 1 - 34) using the following gradient: 0 - 3 min, 50% B, 3 - 30 min 50 -100% B, 30 - 35 min 100% B, 35 - 41 min 100 - 50% B with a flow rate of 2.0 mL/min (A: $ddH_2O$ with 0.1 % formic acid; B: MeOH). Concentrated fractions 10 - 12 were further separated by a second semi-preparative HPLC run over Phenyl-Hexyl column to yield pure barnesin A (1, 1.0 mg, $t_R$ = 25.6 min) with the following gradient: 0 - 5 min 10% D, 5 - 38 min 10 - 75% D, 38 - 40 min 75 - 100% D, 40 - 45 min 100% D, 45 - 50 min 100 - 10% D (C: ACN; D: $NH_4OAc$ 20 mM, pH 7.0) with a flow rate of 2.0 mL/min. The samples were analyzed using analytical HPLC over Phenyl-Hexyl column (250 x 4.6 mm) or Luna C18(2) with the following gradient: 0 - 3 min 50% B, 3 - 30 min 50 - 100% B, 30 - 35 min 100% B, 37 - 40 min 100 - 50% B, 35 - 41 min 50% B (A: $ddH_2O$ with 0.1 % formic acid; B: MeOH) (**1**, $t_R$ = 14,38 min).

**Marfey's reaction:** Barnesin A (**1**, 0.2 mg) was hydrolyzed by 6 N HCl (1.0 mL) at 110 °C overnight (15 h).

HCl was removed using SpeedVac (42 °C) and FDAA (1-fluoro-2,4-dinitrophenyl-5-L-alanine amide, 20 μL, 10 mg/mL in acetone) and $NaHCO_3$ (100 μL, 1 M) were added. The reaction was performed under 80 °C for 10 min. The reaction was quenched by adding of HCl (50 μL, 2 N) and D-tyrosine reference were converted accordingly under the same condition. After centrifugation for 15 min at 13,000 rpm the supernatant was analyzed by LC-MS. 1 μL of the reaction was injected and analyzed using the following gradient: 0 -1 min 10 % D, 1- 7 min 70 % D, 7-10 100 % D, 10 - 13.5 min 10 % D (B: ddH2O with 0.1 % formic acid; D: MeCN with 0.1 % formic acid) at a flow rate of 0.7 mL/min.

**Barnesin A (1)**: colourless solid; $[\alpha]_D^{25} - 5.76$ (*c* 0.0910, MeOH); UV (MeOH): $\lambda_{max}$ 196; 214 nm; IR (ATR) $\nu_{max}$: 1240, 1385, 1455, 1545, 1655, 2365, 2860, 2930, 3070, 3260 $cm^{-1}$; ESI-HRMS: *m*/*z*: 488.2864 $[M+H]^+$; calcd. 488.2867.

**Structure assignment:** The molecular formula of barnesin A (1) was assigned as $C_{25}H_{37}O_5N_5$ based on ESI-HRMS analysis (m/z 488.2864 $[M+H]^+$, calcd. 488.2867, Δ = -0.69 ppm) and confirmed by the observation of 25 carbon signals from $^{13}C$-NMR spectrum, including 11 olefinic or aromatic carbons, between $\delta_C$ 114.8 ppm and $\delta_C$ 157.4 ppm, three carbonyl carbons at $\delta_C$ 164.7 ppm, 171.1 ppm and 171.4 ppm, three nitrogen-bearing carbons ($\delta_C$ 39.9 ppm, 48.6 ppm, and 54.6 ppm), and eight aliphatic carbons between $\delta_C$ 13.9 ppm and 37.4 ppm. Detailed analysis of the obtained 2D NMR spectra (DMSO-$d_6$) revealed the structure of a modified di-peptides consisting of a tyrosine moiety, a modified arginine moiety and a fatty acid tail. The di-peptide was identified based on the presence of two α-carbons ($\delta_H$ 4.47 ppm/$\delta_C$ 54.6 ppm and $\delta_H$ 4.34 ppm/$\delta_C$ 48.6 ppm). The tyrosine moiety was assigned based on COSY correlations of *N*H(3) ($\delta_H$ 8.02 ppm) to H-10 ($\delta_H$ 4.47 ppm) to H-11 ($\delta_H$ 2.87/2.56 ppm, $\delta_C$ 37.4 ppm), and correlations of the *para*-substituted aromatic protons H-13 ($\delta_H$ 6.99 ppm/$\delta_C$ 130.0 ppm) to H-14 ($\delta_H$ 6.60 ppm/$\delta_C$ 114.8 ppm). This assignment was confirmed by the HMBC correlations of H-10 to C-9/C-11/C-12, H-11 to C-9/C-10/C-12/C-13, H-13 to C-11/C-14/C-15 and H-14 to C-12/C-15. Furthermore, two unsaturated *(trans, J* = 15.4 Hz and 15.7 Hz. respectively) spin systems were observed, one of which correlated to an α,β-unsaturated carbonyl group of a modified γ-amino acid connected to the tyrosine moiety (COSY correlation of *N*H(2) to H-4 and HMBC correlation of *N*H(2) to C-9). This was supported by COSY correlations from H-2 to H-3 to H-4 to H-5 to H-6 to H-7 and *N*H(1), and corresponding HMBC correlations of H-2/H-3 to C-1 ($\delta_C$ 171.4 ppm). The presence of guanidine moiety was deduced based on the chemical shift of quaternary carbon at C-8 ($\delta_C$ 157.4 ppm) together with the requirement of molecular formula and unsaturation degree, as well as HMBC correlation of H-7 to C-8. The second unsaturated spin system belonged to an unsaturated fatty acid moiety (COSY correlations from olefinic proton H-17 ($\delta_H$ 5.89 ppm/$\delta_C$ 124.3 ppm) to H-18 ($\delta_H$ 6.49 ppm/$\delta_C$ 142.6 ppm) and to methylene protons H-19, H-20, H-21, H-22, and the presence of a methyl group ($\delta_H$ 0.86 ppm/$\delta_C$ 13.9 ppm). HMBC correlations of H-17/H-18/*N*H(3) to C-16 revealed the connection to the N-terminus of assigned tyrosine moiety. The structure assignment was further confirmed by ESI-HR-MS/MS fragmentation, which revealed the fragment ion pair of *m*/*z* at 125.0964 and 364.1966 corresponding to the amine bond cleavage on the N-terminal of tyrosine moiety. The ion pair of *m*/*z* at 288.1583 and 201.1339 indicated the amine bond cleavage on the C-terminal of tyrosine moiety.

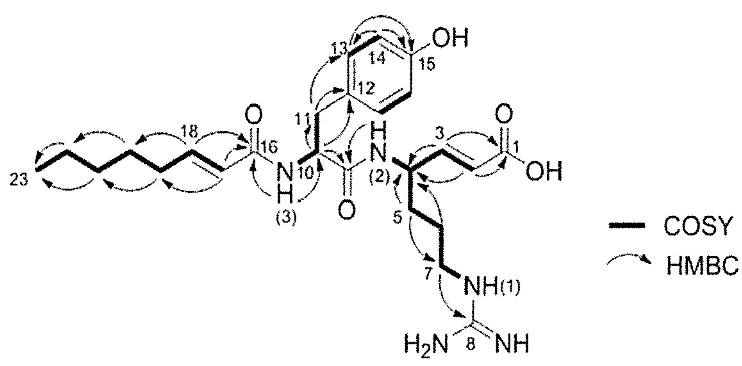


1

In summary, on the basis of the obtained NMR data, the compound was named according to its producing organism: barnesin A (1).

**Table 1.** NMR data of barnesin A (1) (DMSO-$d_6$, at 300 K)[a].

| Position | $\delta_C$, type[b] | $\delta_H$, mult. (*J* in Hz) | COSY | HMBC | NOESY |
|---|---|---|---|---|---|
| 1 | 171.4, qC | | | | |

(continued)

| Position | $\delta_C$, type[b] | $\delta_H$, mult. (*J* in Hz) | COSY | HMBC | NOESY |
|---|---|---|---|---|---|
| 2 | 127.8, CH | 5.73, d (15.7) | 3 | 1, 4 | 3, 4, 10, 11b, *N*H(2) |
| 3 | 141.2, CH | 6.42, dd (15.7, 5.0) | 2,4 | 1, 2, 4 | 2, 4, 5b, 6, *N*H(2) |
| 4 | 48.6, CH | 4.34, br s | 3, 5a, 5b, *N*H (2) | | 2, 3, 5b, 6, 7a, 7b, *N*H(2) |
| 5a | 30.7, $CH_2$ | 1.36, m | 4, 5b | 4, 7 | 3, 4, 5b, 7a, *N*H(2) |
| 5b | | 1.68, m | 4, 5a, 6 | | 3, 4, 6, 7a, *N*H(2) |
| 6 | 24.9, $CH_2$ | 1.46, m | 5b, 7a, 7b | 4, 5, 7 | 3, 4, 5b, 7a, NH(2) |
| 7a | 39.9, $CH_2$ | 3.05, m | 6 | 8 | 4, 6, 5b |
| 7b | | 3.09, m | 6 | | 4, 6, 5b |
| 8 | 157.4, qC | | | | |
| 9 | 171.1, qC | | | | |
| 10 | 54.6, CH | 4.47, t (8.4) | 11a, 11b, *N*H (3) | 9, 11, 12 | 6, 10, 11a, 11b, 13, *N*H(2), *N*H(3) |
| 11a | 37.4, $CH_2$ | 2.56, t (12.0) | 10, 11b | 9, 10, 12, 13 | 10, 11b, 13, *N*H(2), *N*H(3) |
| 11b | | 2.87, d (12.0) | 10, 11a | 9, 10, 12, 13 | 2, 10, 11a, 13, *NH(2)*, *N*H(3) |
| 12 | 128.1, qC | | | | |
| 13 | 130.0, CH | 6.99, d (8.0) | 14 | 11, 14, 15 | 2, 10, 11a, 11b, 14, *N*H(1), *N*H(3) |
| 14 | 114.8, CH | 6.60, d (8.0) | 13 | 12, 15 | 13 |
| 15 | 155.7, qC | | | | |
| 16 | 164.7, qC | | | | |
| 17 | 124.3, CH | 5.89, d (15.4) | 18 | 16, 19 | 18, 19, *N*H (3) |
| 18 | 142.6, CH | 6.49, dd (15.4, 6.7) | 17, 19 | 16, 17, 19, 20 | 17, 19 |
| 19 | 31.1, $CH_2$ | 2.07, m | 18, 20 | 17, 18, 20, 21 | 17, 18 |
| 20 | 27.5, $CH_2$ | 1.36, m | 19, 21 | 18, 19, 22 | |
| 21 | 30.8, $CH_2$ | 1.24, m | 20, 22 | 19, 20, 22, 23 | |

(continued)

| Position | $\delta_C$, type[b] | $\delta_H$, mult. (*J* in Hz) | COSY | HMBC | NOESY |
|---|---|---|---|---|---|
| 22 | 21.9, $CH_2$ | 1.27, m | 21, 23 | 20, 21, 23 | |
| 23 | 13.9, $CH_3$ | 0.86, t (8.6) | 22 | 21, 22 | |
| *N*H(1) | | 10.09, br s | 7a, 7b | | |
| *N*H2) | | 8.50, d (8.1) | 4 | 9 | 2, 3, 4, 5b, 6, 7a, 10, 11a, 11b, 13, *N*H (3) |
| *N*H(3) | | 8.02, d (8.4) | 10 | 10, 11, 16 | 10, 11a, 11b, 13, 17, *N*H(3) |
| [a] 600 MHz for $^1$H NMR, COSY, HSQC and HMBC; 150 MHz for $^{13}$C NMR; 500 MHz for NOESY; [b] numbers of attached protons were determined by analysis of 2D spectra | | | | | |

**Example 2 Synthesis according to Reaction Scheme 1**

Specific examples for the preparation of compounds of formula (I) are provided below. Unless otherwise specified all starting materials and reagents are of standard commercial grade, and are used without further purification, or are readily prepared from such materials by routine methods. Those skilled in the art of organic synthesis will recognize that starting materials and reaction conditions may be varied including additional steps employed to produce compounds encompassed by the present invention.

***Reaction Scheme 3***

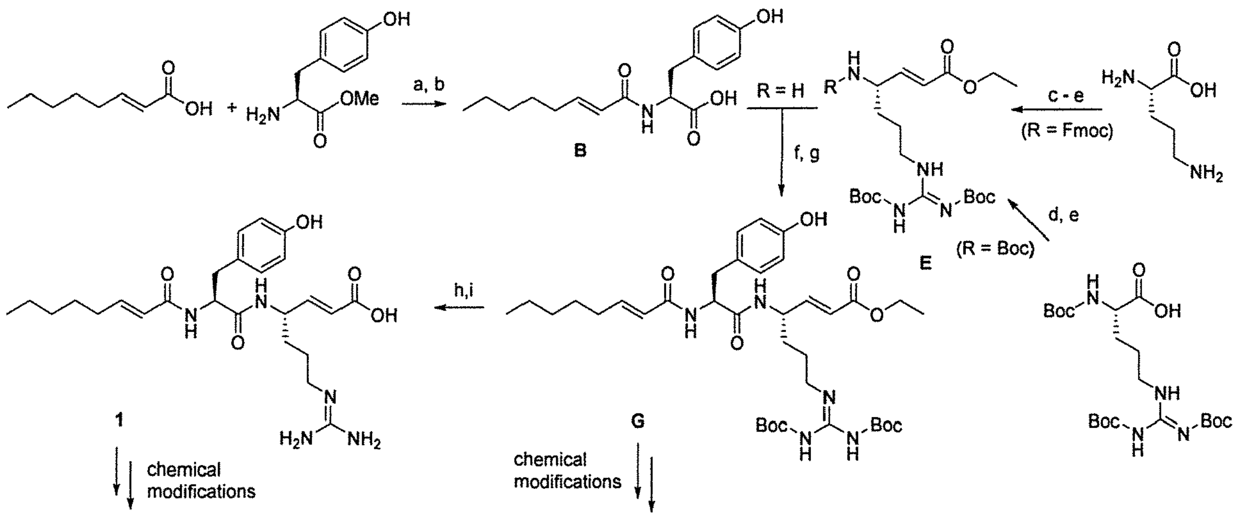


a) HBTU, DIPEA, DCM, r.t., o.n., 78%; b) 10%-$_{aq}$ KOH, MeOH, r.t., 3 h, quant.; c) 1. $CuCO_3 \cdot Cu(OH)_2$, $H_2O$, r.t., 15 min, quant; 2. bis-Boc-pyrazolocarboxamidine, DIPEA, formamide, dioxane, r.t., o.n., 3. EDTA·2Na·2$H_2O$, $NaHCO_3$, $H_2O$ then Fmoc-OSu, acetone, r.t., o.n. 70% (three steps); d) N,O-dimethylhydroxylamin hydrochlorid, HBTU, DIPEA, DCM, r.t., o.n. 71%; e) 1. $LiAlH_4$, THF, 0 °C, 30 min, 2. triethyl phosphonoacetate, NaH, THF, 0 °C, 45 min, 32% over two steps; f) 20% piperidine in DMF, r.t., 4 h, 86%; g) COMU, DIPEA, DMF, 0 °C, 6 h, 27%; h) TFA, DCM, 0 °C to r.t., o.n.; i) porcine liver esterase, DMSO, $H_2O$, Tris/HCl buffer, 37 °C, 5 d, 47% over two steps.

***2.1. Methyl (E)-oct-2-enoyl-tyrosinate (A)***

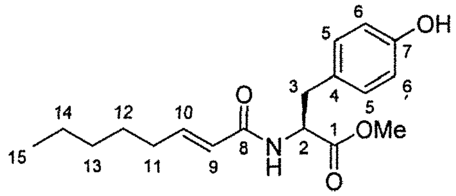

**A**

To a solution of *trans*-2-octenoic acid (500 mg, 3.52 mmol) in DCM (3.5 mL) was added subsequently DIPEA (1.5 mL, 1.14 g, 8.79 mmol) and HBTU (1.60 g, 4.22 mmol). The mixture was stirred for 5 min at room temperature (r.t.) followed by the addition of H-Tyr-OMe HCl (896 mg, 3.87 mmol). The reaction was stirred at r.t. overnight. During this time the colourless suspension became a yellowish solution. The reaction was quenched by adding 10% aq. citric acid solution to the reaction mixture. The suspension was extracted three times with DCM. The combined organic layers were washed with sat. $NaHCO_3$ and brine, dried over $MgSO_4$, filtered and evaporated. The crude product was purified by flash chromatography over a silica gel column (mobile phase: cyclohexane/EtOAc 1:0 to 1:1). The appropriate fractions were collected and evaporated to afford ester A (878 mg, 2.75 mmol, 78% yield) as yellowish oil.

**$^1$H-NMR (600 MHz, $CDCl_3$):** $\delta$ = 6.94-6.93 (m, 2H, 5-H), 6.85 (dt, *J* = 15.3, 6.9 Hz, 1H, 10-H), 6.74- 6.72 (m, 2H, 6-H), 5.96 (d, *J* = 7.9 Hz, 1H, *N*H), 5.77 (d*, J = 15.3 Hz, 1H, 9-H, *fine splitting), 4.93 (dt, *J* = 7.9, 5.7 Hz, 1H, 2-H), 3.73 (s, 3H, OMe), 3.17 (dd, *J* = 14.0, 5.7 Hz, 1H, 3-H), 3.04 (dd, *J* = 14.0, 5.7 Hz, 1H, 3-H), 2.16 (q*, *J* = 6.9 Hz, 2H, 11-H, *fine splitting), 1.43 (quint, *J* = 7.3 Hz, 2H, 12-H), 1.32 - 1.25 (m, 4H, 13-H, 14-H), 0.88 (t, *J* = 7.0 Hz, 3H, 15-H) ppm.

**$^{13}$C-NMR (150 MHz, $CDCl_3$):** $\delta$ = 172.3, 165.7, 155.2 (3s, C-1, C-8, C-7), 146.4 (d, C-10), 130.3 (d, 2xC-5), 127.3 (s, C-4), 122.7 (d, C-9), 115.5 (d, 2xC-6), 53.3 (d, C-2), 52.4 (q, OMe), 37.2, 32.0, 31.3, 27.8, 22.4 (5t, C-3, C-11, C-13, C-12, C-14), 14.0 (q, C-15) ppm.

**HRMS (ESI-TOF):** calculated for $C_{18}H_{26}NO_4$ $[M + H]^+$: 320.1856; found 320.1859.

***2.2. (E)-Oct-2-enoyl-tyrosine (B)***

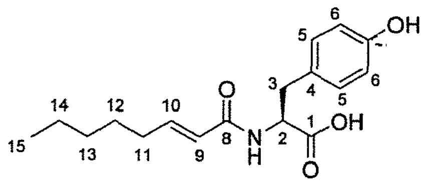

**B**

To a solution of A (81 mg, 0.25 mmol) in MeOH (1.0 mL) was added 10% aq. KOH (250 $\mu$l) and the mixture was stirred for 3 h at r.t. The reaction was acidified by adding 1M HCl and then extracted with EtOAc twice. The combined organic layers were washed with water and brine, dried over $MgSO_4$, filtered and evaporated to afford acid **B** (79 mg, crude product) as clear oil. The crude product was used in the next step without further purification.

**$^1$H-NMR (600 MHz, MeOD-*d$_4$*):** $\delta$ = 7.04-7.02 (m, 2H, 5-H), 6.74 (dt, *J* = 15.4, 7.1 Hz, 1H, 10-H), 6.69-6.68 (m, 2H, 6-H), 5.95 (d*, *J* = 15.4 Hz, 1H, 9-H, *fine splitting), 4.65 (dd, *J* = 8.7, 5.2 Hz, 1H, 2-H), 3.11 (dd, *J* = 14.0, 5.2 Hz, 1H, 3-H), 2.89 (dd, *J* = 14.0, 8.7 Hz, 1H, 3-H), 2.18 (q*, *J* = 7.0 Hz, 2H, 11-H, *fine splitting), 1.45 (quint, *J* = 7.3 Hz, 2H, 12-H), 1.36 - 1.29 (m, 4H, 13-H, 14-H), 0.91 (t, *J* = 7.0 Hz, 3H, 15-H) ppm.

**$^{13}$C-NMR (150 MHz, MeOD-*d$_4$*):** $\delta$ = 174.9, 168.5, 157.3 (3s, C-1, C-8, C-7), 146.4 (d, C-10), 131.2 (d, 2xC-5), 129.1 (s, C-4), 124.2 (d, C-9), 116.2 (d, 2xC-6), 55.4 (d, C-2), 37.7, 33.0 (t, C-11), 32.5, 29.1, 23.5 (5t, C-3, C-11, C-13, C-12, C-14), 14.3 (q, C-15) ppm.

**HRMS (ESI-TOF):** calculated for $C_{17}H_{24}NO_4$ $[M + H]^+$: 306.1700; found 306.1702.

***2.3. Fmoc-Arg-(Boc)$_2$-OH (C)***

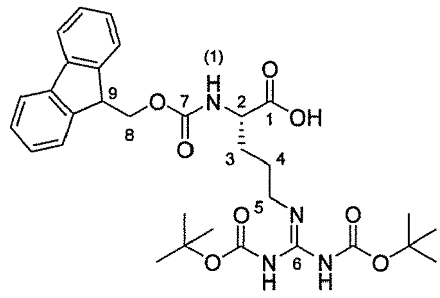

**C**

A mixture containing ornithine hydrochloride **5** (1.0 g, 5.93 mmol) and basic copper carbonate $CuCO_3$ (656 mg, 2.97 mmol) dissolved in dest. $H_2O$ (8 mL) was stirred under reflux for 15 min. The blue reaction mixture was filtered, evaporated and dried under vacuum to afford a blue solid (1.22 g, quant). Part of the blue solid (600 mg) was suspended in formamide (6 mL) and DIPEA (1.2 mL, 880 mg, 6.81 mmol) was added. To the blue solution was added drop wise bis-boc-pyrazolocarboxamidine (939 mg, 3.02 mmol) in dioxane (3 mL). The blue solution was stirred at r.t. overnight, and then quenched with $H_2O$ (20 mL) to yield a light blue solid precipitate. The suspension was extracted twice with EtOAc. The combined organic layers were washed with brine, dried over $MgSO_4$, filtered and evaporated to yield blue foam (1.48 g). The crude product was suspended in $H_2O$ (6 mL) and EDTA·2Na·$2H_2O$ (676 mg, 1.82 mmol) and sat. aq. $NaHCO_3$ (597 mg, 7.11 mmol) were added, then 9-fluorenylmethyl-succinimidyl carbonate (1.22 g, 3.63 mmol), dissolved in acetone (13.5 mL), was added drop wise to the reaction mixture at r.t. The blue suspension turned light blue and the reaction mixture was stirred at r.t. overnight. Subsequently, the solvent was evaporated under vacuum, and $H_2O$ (25 mL) was added resulting in a slight basic light blue turbid mixture (pH 8). The mixture was acidified by adding 10% aq. citric acid solution (pH 2-3). The resulting solution was extracted twice with EtOAc. The combined organic layers were washed with water and brine, dried over $MgSO_4$, filtered and evaporated. The crude product was purified by chromatography over a silica gel column (mobile phase: cyclohexane/EtOAc + 1% AcOH 10:1 to 1:1). The appropriate fractions were collected and evaporated to afford triprotected arginine C (1.22 g, 2.05 mmol, 70% yield over two steps) as colorless foam.

**$^1$H-NMR (600 MHz, $CDCl_3$):** $\delta$ = 11.37 (br. s, 1H, $NHCO_2$*t*Bu), 8.50, 8.42* (s, 1H, $NHCO_2$*t*Bu), 7.75-7.74 (m, 2H, Ar), 7.62-7.59, 7.56* (m, 2H, Ar), 7.39-7.37 (m, 2H, Ar), 7.31-7. 28 (m, 2H, Ar), 5.89*, 5.83 (d, *J*= 8.1 Hz, *N*H(1)), 4.62*, 4.52*, 4.42-4.37 (m, 3H, 2-H, 8-H), 4.21 (t, *J*= 7.0 Hz, 1H, 9-H), 3.46-3.39, 3.32* (m, 2H, 5-H), 1.98-1.94 (m, 1H, 3-H), 1.76-1.66 (m, 3H, 3-H, 4-H), 1.48 (s, 9H, $NHCO_2$*t*Bu), 1.47 (s, 9H, $NHCO_2$*t*Bu) ppm *minor rotamer.

**$^{13}$C-NMR (150 MHz, $CDCl_3$):** $\delta$ = 174.9, 172.5* (s, C-1), 163.3*, 162.8 (s, C-6), 156.6*, 156.4 (s, C-7), 156.4 (s, $NHCO_2$*t*Bu), 153.3, 152.9* (s, $NHCO_2$*t*Bu), 144.0*, 143.8 (2s, Ar), 141.4 (s, 2Ar), 127.8 (d, 2Ar), 127.2 (d, 2Ar), 125.3, 124.9* (d, Ar), 120.1 (d, 2Ar), 84.3*, 83.7, 83.4* (2s, $NHCO_2$*t*Bu), 80.5*, 80.1, 79.7* (2s, $NHCO_2$*t*Bu), 67.3*, 67.1 (t, C-8), 54.1*, 53.7 (d, C-2), 47.3 (d, C-9), 40.6, 40.4*
(t, C-5), 29.8*, 29.7 (t, C-3), 28.4*, 28.3 (q, $NHCO_2$*t*Bu), 28.2*, 28.2 (q, $NHCO_2$*t*Bu), 25.5, 25.3* (t, C-4) ppm *minor rotamer.
**HRMS (ESI-TOF):** calculated for $C_{31}H_{41}N_4O_8$ $[M + H]^+$ 597.2919; found 597.2924.

### *2.4. N1-Fmoc-N3,N4-di-Boc-arginine Weinreb amide (D)*

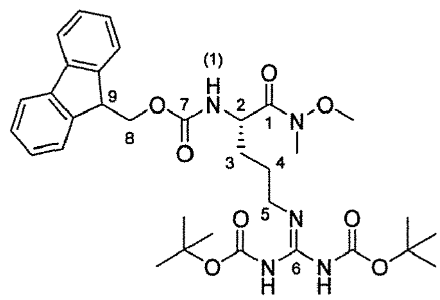

**D**

To a solution of C (300 mg, 0.503 mmol) in DCM (5 mL) were added subsequently DIPEA (263 μl, 195 mg, 1.51 mmol) and HBTU (229 mg, 0.603 mmol). The mixture was stirred at r.t. for 5 min followed by the addition of N,O-dimethylhydroxylamine hydrochlorid (98 mg, 1.01 mmol). The reaction was stirred at r.t. overnight. To the reaction mixture was added $H_2O$ and the resulting suspension was extracted twice with EtOAc. The combined organic layers were washed with brine, dried over $Na_2SO_4$, filtered and evaporated. The crude product was purified by flash chromatography over a silica column (mobile phase: cyclohexane/EtOAc 1:0 to 1:1). The appropriate fractions were collected and evaporated to afford weinreb amide **D** (228 mg, 0.356 mmol, 71% yield) as colorless foam.

**$^1$H-NMR (500 MHz, $CDCl_3$)**: δ = 11.50 (s, 1H, N<u>H</u>CO$_2$tBu), 8.40, 8.28* (s, 1H, N<u>H</u>CO$_2$tBu), 7.77-7.75 (m, 2H, Ar), 7.62-7.60, 7.57* (m, 2H, Ar), 7.41-7.38 (m, 2H, Ar), 7.33-7.30 (m, 2H, Ar), 5.61, 5.15* (d, *J* = 8.9 Hz, 1H, *N*<u>H</u>(1)), 4.78-4.70, 4.60* (m, 1H, 2-H), 4.39 (dd, *J* = 10.5, 7.5 Hz, 1H, 8-H), 4.35 (dd, *J* = 10.5, 7.0 Hz, 1H, 8-H), 4.22 (t, *J*= 7.0 Hz, 1H, 9-H), 3.77 (s, 3H, N(CH$_3$)OCH$_3$), 3.47 - 3.46, 3.36* (m, 2H, 5-H), 3.22, 3.11* (s, 3H, N(CH$_3$)OCH$_3$), 1.84-1.80 (m, 1H, 3-H), 1.69-1.62 (m, 3H, 3-H, 4-H), 1.50 (s, 9H, NHCO$_2$*t*<u>Bu</u>), 1.49 (s, 9H, NHCO$_2$<u>tBu</u>) ppm *minor rotamer.

**$^{13}$C-NMR (125 MHz, $CDCl_3$):** δ = 172.2, 163.1, 156.1 (3s, C-1, C-6, C-7), 156.0, 153.2 (2s, NH<u>C</u>O$_2$tBu), 143.9*, 143.8 (s, 2xAr), 141.3*, 141.3 (s, 2Ar), 127.6 (d, 2xAr), 127.0 (d, 2xAr), 125.2*, 125.1 (d, 2xAr), 119.9*, 119.9 (d, 2xAr), 83.3, 79.5 (2s, NHCO$_2$*t*<u>Bu</u>), 67.0 (t, C-8), 61.6 (q, N(CH$_3$)OCH$_3$), 50.7, 47.2 (2d, C-2, C-9), 40.5 (t, C-5), 32.1 (q, N(CH$_3$)OCH$_3$), 29.9 (t, C-3), 28.3, 28.0 (2q, NHCO$_2$*t*<u>Bu</u>), 25.1 (t, C-4) ppm *minor rotamer.

**HRMS (ESI-TOF):** calculated for $C_{33}H_{46}N_5O_8$ $[M + H]^+$ 640.3341; found 640.3347.

### *2.5. N1-Fmoc-N3,N4-di-Boc-vinylogous arginine ethyl ester (E)*

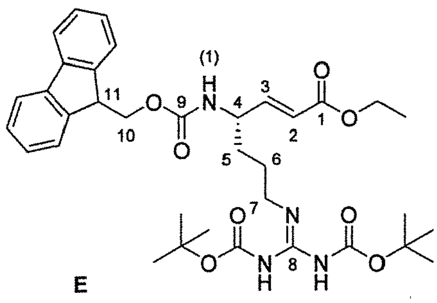

E

A solution of weinrebamide **D** (221 mg, 0.345 mmol) in dry THF (3.5 mL) was cooled to 0 °C followed by slow addition of $LiAlH_4$ (66 mg, 1.73 mmol). The mixture was stirred for 30 min at 0 °C. The reaction was quenched with 10% aq. citric acid solution and extracted twice with EtOAc. The combined organic layers were washed with water and brine, dried over $MgSO_4$, filtered. The solvent was removed under reduced pressure to afford the aldehyde (146 mg) as colorless foam which was used in the next step without further purification.

To an ice-cooled solution of triethyl phosphonoacetate (60 μl, 68 mg, 0.302 mmol) in dry THF (0.5 mL) was added NaH (15 mg, 0.377 mmol, 60% in mineral oil). After 30 min of stirring at 0 °C, the crude aldehyde (146 mg) dissolved in dry THF (2 mL) was added drop wise at 0 °C to the reaction mixture. Upon completed addition, the reaction was stirred for 45 min at 0 °C. The mixture was poured into 10% aq. citric acid solution and the suspension was extracted twice with EtOAc. The combined organic layers were washed with brine, dried over $MgSO_4$, filtered and evaporated. The crude product was purified by chromatography over a silica gel column (mobile phase: cyclohexane/EtOAc 5:1). The appropriate fractions were collected and evaporated to afford protected vinyl arginine E (71 mg, 0.109 mmol, 32% yield over two steps) as colorless oil.

**$^1$H-NMR (300 MHz, $CDCl_3$):** δ = 11.52 (s, 1H, N<u>H</u>CO$_2$tBu), 8.39 (m, 1H, N<u>H</u>CO$_2$tBu), 7.76-7.73 (m, 2H, Ar), 7.66-7.59 (m, 2H, Ar), 7.40-7.35 (m, 2H, Ar), 7.32-7.25 (m, 2H, Ar), 6.84 (dd, J= 15.6 Hz, 5.0 Hz, 1H, 3-H), 6.05 (d, *J* = 8.9 Hz, 1H, *N*<u>H</u>(1)), 5.98 (d, *J*= 15.6 Hz, 1H, 2-H), 4.44-4.41 (m, 3H, 4-H, 10-H), 4.22-4.14 (q, J = 7.1 Hz, 3H, OC<u>H</u>$_2$CH$_3$, 11-H), 3.58-3.53 (m, 1H, 7-H), 3.32-3.26 (m, 1H, 7-H), 1.66 - 1.58 (m, 4H, 5-H, 6-H), 1.49 (s, 18H, 2 NHCO$_2$t<u>Bu</u>), 1.28 (t, *J* = 7.1 Hz, 3H, OCH$_2$C<u>H</u>$_3$) ppm.

**$^{13}$C-NMR (75 MHz, $CDCl_3$):** δ = 166.3, 163.5, 156.6 (3s, C-1, C-8, C-9), 156.0, 153.4 (2s, NH<u>C</u>O$_2$tBu), 148.0 (d, C-3), 144.1, 143.8, 141.4, 141.3 (4s, Ar), 127.7 (d, 2xAr), 127.1 (d, 2xAr), 125.3, 125.1 (2d, Ar), 121.1 (d, C-2), 120.0 (d, 2xAr), 83.3, 79.5 (2s, NHCO$_2$<u>tBu</u>), 66.6 (t, C-10), 60.5 (t, O<u>C</u>H$_2$CH$_3$), 52.3, 47.4 (2d, C-4, C-11), 40.3 (t, C-7), 30.2, 29.7* (t, C-5), 28.3, 28.1 (2q, NHCO$_2$<u>tBu</u>), 27.0*, 26.2 (t, C-6), 14.3 (q, OCH$_2$<u>C</u>H$_3$) ppm *minor rotamer.

**HRMS (ESI-TOF):** calculated for $C_{35}H_{47}N_4O_8$ $[M + H]^+$ 651.3388; found 651.3400.

### 2.6. N3,N4-Di-Boc-vinylogous arginine ethyl ester (F)

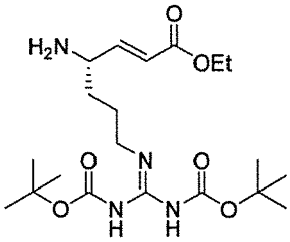

**F**

Compound E (71 mg, 0.109 mmol) was dissolved in DMF (1 mL) and piperidine (200 μl) was added. The solution was stirred at r.t. for 4 h. The reaction was evaporated and dried under vacuum overnight. The crude product was purified by chromatography over a silica gel column (mobile phase gradient: from cyclohexane/EtOAc 1:1 to 0:1, switched to DCM/MeOH 9:1). The appropriate fractions were collected and evaporated to afford amine **F** (40 mg, 0.093 mmol, 86% yield) as colorless oil.

**$^1$H-NMR (300 MHz, MeOD-*d$_4$*):** δ = 6.88 (dd, *J*= 15.7 Hz, 6.7 Hz, 1H, 3-H), 5.98 (dd, *J*= 15.7 Hz, 1.2 Hz, 1H, 2-H), 4.18 (q, J= 7.1 Hz, 2H, $OCH_2CH_3$), 3.53-3.47 (m, 1H, 4-H), 3.40 - 3.35 (m, 2H, 7-H), 1.68-1.57 (m, 4H, 5-H, 6-H), 1.52 (s, 9H, $NHCO_2$*tBu*), 1.47 (s, 9H, $NHCO_2$*tBu*), 1.28 (t, *J*= 7.1 Hz, 3H, $OCH_2CH_3$) ppm.

**$^{13}$C-NMR (75 MHz, MeOD-*d$_4$*):** δ = 168.1, 164.5 (2s, C-1, C-8), 157.6, 154.1 (2s, $NCO_2$*t*Bu), 152.7, 121.5 (2d, C-3, C-2), 84.4, 80.3 (2s, $NHCO_2$*tBu*), 61.5 (t, $OCH_2CH_3$), 53.2 (d, C-4), 41.4, 34.5 (2t, C-7, C-5), 28.6, 28.2 (2q, $NHCO_2$*tBu*), 26.5 (t, C-6), 14.5 (q, $OCH_2CH_3$) ppm.

**HRMS (ESI-TOF):** calculated for $C_{20}H_{37}N_4O_6$ $[M + H]^+$: 429.2708; found 429.2713.

### 2.7. (E)-Oct-2-enoyl-tyrositte-N3,N4-di-Boc-vinylogous arginine ethyl ester (G)

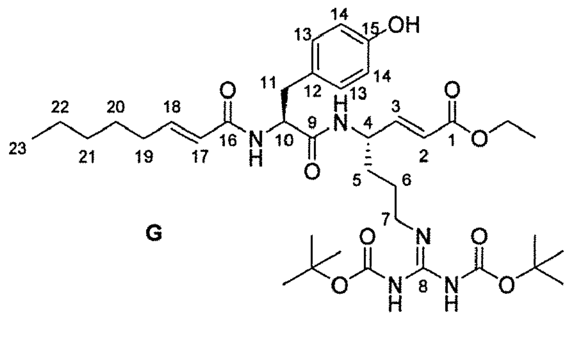

**G**

To a solution of amine F (40 mg, 0.093 mmol) and octenoic acid (43 mg, 0.140 mmol) in DMF (1.0 mL) was added DIPEA (81 μl, 60 mg, 0.467 mmol) (pH > 7). The mixture was cooled to 0 °C followed by the addition of COMU (64 mg, 0.149 mmol). The solution was stirred for 6 h at this temperature. The reaction was poured into 10% aq. citric acid solution and the suspension was extracted with EtOAc twice and three times with a DCM/IPA (4:1) mixture. The combined organic layers were washed with $H_2O$ and brine, dried over $Na_2SO_4$, filtered and evaporated. The crude product was purified by HPLC using a semipreparative phenyl hexyl column (mobile phase: A: 20 mM aq. $NH_4OAc$, pH 7, B: MeOH gradient: 80% B 5 min, from 80% to 97% B in 30 min, 97% B 15 min). The appropriate fractions were collected and evaporated to afford compound G (18 mg, 0.025 mmol, 27% yield) as yellowish oil.

**$^1$H-NMR (300 MHz, MeOD-*d$_4$*):** δ = 7.05 - 7.02 (m, 2H, 13-H), 6.82 - 6.66 (m, 4H, 3-H, 14-H, 18-H), 5.97 (dt, *J* = 15.4 Hz, 1.5 Hz, 1H, 17-H), 5.64 (dd, *J* = 15.7 Hz, 1.5 Hz, 1H, 2-H), 4.60 - 4.55 (m, 1H, 10-H), 4.52 - 4.47 (m, 1H, 4-H), 4.18 (q, *J*= 7.0 Hz, 2H, $OCH_2CH_3$), 3.39 - 3.33 (m, 2H, 7-H), 2.97 (dd, *J* = 13.5 Hz, 8.0 Hz, 1H, 11-H), 2.84 (dd, *J* = 13.5 Hz, 7.3 Hz, 1H, 11-H), 2.18 (q*, *J* = 7.1 Hz, 2H, 19-H, *fine splitting), 1.67 - 1.53 (m, 4H, 5-H, 6-H), 1.52 (s, 9H, $NHCO_2$*tBu*), 1.46 (s, 9H, $NHCO_2$*tBu*), 1.45 - 1.41 (m, 2H, 20-H), 1.36 - 1.25 (m, 4H, 21-H & 22-H), 1.29 (t, *J* = 7.0 Hz, 3H, $OCH_2CH_3$), 0.91 (t, *J* = 7.0 Hz, 3H, 23-H) ppm.

**$^{13}$C-NMR (75 MHz, MeOD-$d_4$):** $\delta$ = 173.4, 168.3, 167.9, 164.6 (4s, C-9, C-16, C-1, C-8), 157.6 (s, $NHCO_2tBu$), 157.4 (s, C-15), 154.1 (s, $NHCO_2tBu$), 148.8, 146.4 (2d, C-3, C-18), 131.3 (d, 2xC-13), 128.6 (s, C-12), 124.3, 122.0 (2d, C-17, C-2), 116.3 (d, 2xC-14), 84.4, 80.3 (2s, NHCO2tBu), 61.6 (t, $OCH_2CH_3$), 56.7, 51.2 (2d, C-10, C-4), 41.3, 38.5, 33.0, 32.5, 31.9, 29.1 (6t, C-7, C-11, C-19, C-21, C-5, C-20), 28.6, 28.3 (q, $NHCO_2tBu$), 26.7 (t, C-6), 23.5 (2q, NHCO2tBu), 14.6 (q, $OCH_2CH_3$), 14.4 (q, C-23) ppm.

### *2.8. (E)-Oct-2-enoyl-tyrosine -vinylogous arginine ethyl ester (3)*

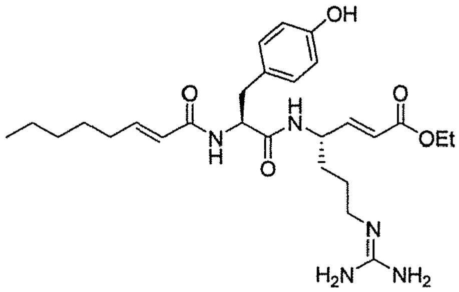

A solution of ester **G** (18 mg, 25.1 µmol) in DCM (950 µl) was treated with TFA (50 µl) for 1 hour at 0 °C followed by stirring the reaction for 1.5 h at r.t.. Additional TFA (50 µl) was added and the reaction was stirred at r.t. overnight. The reaction mixture was evaporated and dried under vacuum to afford the ester **3** as yellowish oil. The crude product was used in the next step without further purification.
**$^1$H-NMR (500 MHz, MeOD-d4):** $\delta$ = 7.05 - 7.03 (m, 2H, 13-H), 6.76 (dt, *J*= 15.4 Hz, 7.0 Hz, 1H, 18-H), 6.70 - 6.68 (m, 2H, 14-H), 6.66 (dd, J = 15.8 Hz, 5.6 Hz, 1H, 3-H), 5.98 (d*, J = 15.4 Hz, 1H, 17-H, *fine splitting), 5.59 (dd, J = 15.8 Hz, 1.5 Hz, 1H, 2-H), 4.49 (m, 2H, 4-H, 10-H), 4.19 (dq, J = 7.1 Hz, 2H, $OCH_2CH_3$), 3. 21 - 3.11 (m, 2H, 7-H), 2.95 (dd, J = 13.6 Hz, 8.3 Hz, 1H, 11-H), 2.87 (dd, J = 13.6 Hz, 7.1 Hz, 1H, 11-H), 2.19 (q*, J = 7.1 Hz, 2H, 19-H, *fine splitting), 1.70 - 1.58 (m, 3H, 5-H, 6-H), 1.57 - 1.52 (m, 1H, 5-H), 1.49 - 1.44 (m, 2H, 20-H), 1.37 -1.31 (m, 4H, 21-H, 22-H), 1.29 (t, J = 7.1 Hz, 3H, $OCH_2CH_3$), 0.91 (t, J = 6.9 Hz, 3H, 23-H) ppm.
**$^{13}$C-NMR (125 MHz, MeOD-d4):** $\delta$ = 173.7, 168.5, 167.9, 158.7, 157.5 (5s, C-9, C-16, C-1, C-8, C15), 148.2, 146.4 (2d, C-3, C-18), 131.3 (d, 2xC-13), 128.5 (s, C-12), 124.3, 122.2 (2d, C17, C-2), 116.4 (d, 2xC-14), 61.7 (t, $OCH_2CH_3$), 57.1, 50.7 (2d, C-10, C-4), 42.0, 38.3, 33.0, 32.5, 32.0, 29.1, 26.1, 23.5 (8t, C-7, C-11, C-19, C-21, C-5, C-20, C-6, C-22), 14.5 (q, $OCH_2CH_3$), 14.3 (q, C-23) ppm.
**HRMS (ESI-TOF):** calculated for $C_{27}H_{41}N_5O_5$ $[M + H]^+$: 516.3180; found 516.3183.

### *2.9. Barnesin A (1)*

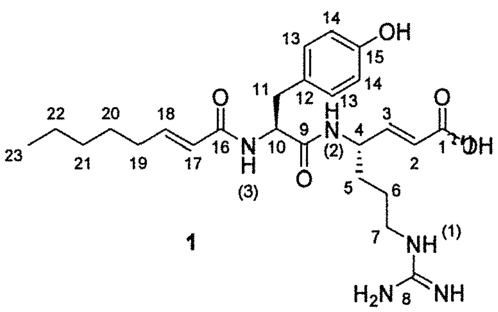

1

Compound 3 was dissolved in DMSO (36 µl) and $H_2O$ (144 µl) followed by the addition of porcine liver esterase (36 mg) in Tris/HCl buffer (1 mL, 50 mM, pH 6.8). The mixture was shaken for 5 days at 37 °C. The reaction was filtered twice over a SPE cartridge. The methanolic and aqueous fractions were combined and evaporated. The crude product was purified by HPLC using a semipreparative phenyl hexyl column (mobile phase: A: 20 mM aq. $NH_4OAc$, pH 7, B: MeOH gradient: 30% B 5 min, from 30% to 80% B in 35 min, 80% B 10 min, from 80% to 95% B in 0.1 min, 95% B 4.9

min). The appropriate fractions were collected and evaporated to afford barnesin A (1) (7.0 mg, 14.4 μmol, 47% yield over two steps, colourless solid).

**HRMS (ESI-TOF):** calculated for $C_{25}H_{38}N_5O_5$ $[M + H]^+$: 488.2867; found 488.2876. $[\alpha]_D^{25} = -5.09°$ (0.0910; MeOH)

**Example 3 Synthesis according to Reaction Scheme 2**

***3.1. Preparation of aldehyde***

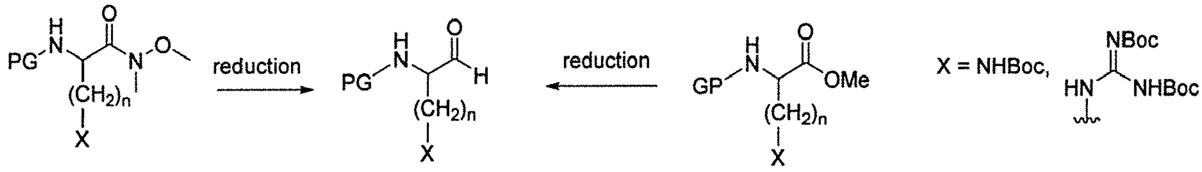


**Weinreb amide route (GPC):** Weinreb amide was solubilized in dry THF (1.0 mmol/mL) and cooled to 0 °C. $LiAlH_4$ (5.0 eq) was added in portions over 30 min and the suspension was stirred at 0 °C for additional 2 h. Reaction control was performed using TLC. In case reduction was incomplete after 2 h, the reaction mixture was warmed up to r.t., $LiAlH_4$ (5.0 - 7.0 eq) was added and the reaction mixture was stirred for further 1-2 h depending on the reaction process. The reaction mixture was quenched using 10% aq. citric acid and extracted twice with EtOAc. The combined organic layers were washed twice with dest. $H_2O$, twice with brine, dried over $MgSO_4$, filtered and concentrated to obtain a yellow foamy oil. The crude product was used without further purification in the Horner-Wasdsworth-Emmons (HWE) reaction (see below).

*Methyl ester* route **(GPD):** Amino acid methyl ester was solubilized in dry DCM (50 μmol/mL) and cooled to -80 °C. DIBAL-(H) (1.2 M in toluene, 2.0 eq) was added dropwise over 1 h and the reaction mixture stirred at -80 °C for one additional hour. Reaction control was performed using TLC. In case reduction was incomplete after one hour, additional 0.5 eq DIBAL-(H) was added every 30 min until full conversion of the methyl ester to the aldehyde. The solution was then quenched with a sat. solution of Rochelle salt until the production of gas ceased. The reaction mixture was extracted with DCM and warmed up at r.t. DCM and dest. $H_2O$ were added in order to get a clear suspension, which was strongly stirred for an additional hour. The aq. phase was at last extracted with DCM, and the combined org. layers were washed with brine, dried over $Na_2SO_4$, filtered and concentrated to obtain a yellowish oil. The crude product was used without further purification in the HWE-step (see below).

3.1.1 Products

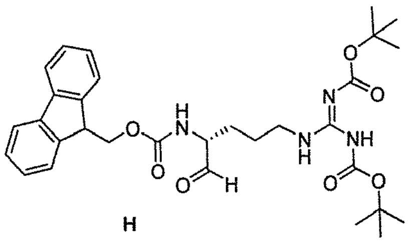

**H**

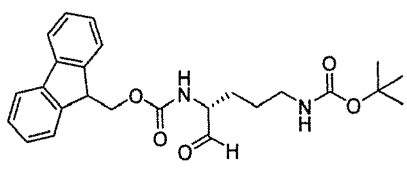

**I**

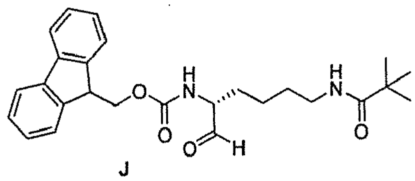

**J**

Fmoc-Arg(ω,ω'-Boc) aldehyde **(H):** According to **GPC,** Fmoc -Arg(ω,ω'-Boc) Weinreb amide (110 mg, 0.17

mmol, 1.0 eq) was converted to aldehyde **(H,** 56 mg) as yellowish oil. According to **GPD,** Fmoc-L-Arg(ω,ω'-Boc)-OMe (720 mg, 1.18 mmol, 1.0 eq) was converted to aldehyde **(H,** 830 mg).

Fmoc-L-Orn(δ-Boc) aldehyde **(I):** According to **GPD,** Fmoc -Orn(δ-Boc)-OMe (340 mg, 0.73 mmol, 1.0 eq) was converted to aldehyde **(I,** 350 mg) as yellowish oil.

Fmoc-L-Lys(ε-Boc) aldehyde (**J**): According to **GPC,** Fmoc- Lys(ε-Boc) Weinreb amide (186 mg, 0.36 mmol, 1.0 eq) was converted to aldehyde **(J.** 126 mg) as yellowish oil. According to **GPD,** Fmoc-Lys(ε-Boc)-OMe (340 mg, 0.70 mmol, 1.0 eq) was converted to aldehyde **(J,** 296 mg) as yellowish oil.

### *3.2. Solide phase peptide synthesis (SPPS)*

3.2.1 Loading of resin

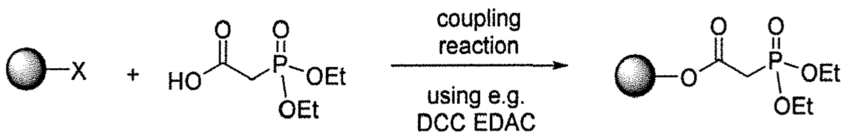


*Wang Resin:* Wang resin (275 mg, 1.40 mmol/g, 0.39 mmol, 1.0 eq) was activated by shaking in DMF (4 mL) for 1 h. The resin was filtrated, cooled to 0 °C and a solution of coupling reagents and diethylphosphonoacetic acid (377 mg, 1.92 mmol, 5.1 eq), dissolved in DMF (2 mL), was added at 0 °C. The coupling reagents were either HBTU (719 mg, 1.93 mmol, 5.0 equ)/HOBt (265 mg, 1.94 mmol, 5.0 eq), or PyBOP (927 mg, 1.92 mmol, 5.1 eq). DIPEA (667 μL, 3.81 mmol, 10.0 eq) was added at the end. The resin was first shaken at 0 °C for 5 min, then at r.t. for additional 24 h. The reaction mixture turned red overtime. Lastly, the resin was filtrated and washed 3 times, alternatively, with DMF (2 mL), and IPA (2 mL). The last wash was with DMF. A capping step was added by shaking the resin a 20% acetanhydrid solution in DMF (2 mL) during 30 min. The resin was then again washed as previously described.

*CTT Resin:* CTT resin (300 mg, 0.875 mmol/g, 0.26 mmol, 1.0 eq) was swelled 1 h, while shaken, in dry DCM (4 mL). A solution of diethylposponoacetic acid (87 mg, 0.44 mmol, 2.5 eq), dissolved in dry DCM (2 mL), was poured on the resin with a first addition of DIPEA (61.1 μL, 0.35 mmol, 2.0 eq). After 5 minutes of shaking, at room temperature, DIPEA (91.7 μL, 0.53 mmol, 3.0 eq) was added a second time. The resin was them shaken at room temperature, overnight. The reaction mixture got yellow overtime. Lastly, the resin was capped by adding MeOH (300 μL), and shaken for further 30 minutes. The resin was filtrated and subsequently washed with DCM (2 mL), DMF (2 mL), DCM (2 mL) and, at the end with MeOH (2 mL).

3.2.2. Horner-Wasdsworth-Emmons reaction on solid phase **(GPE)**

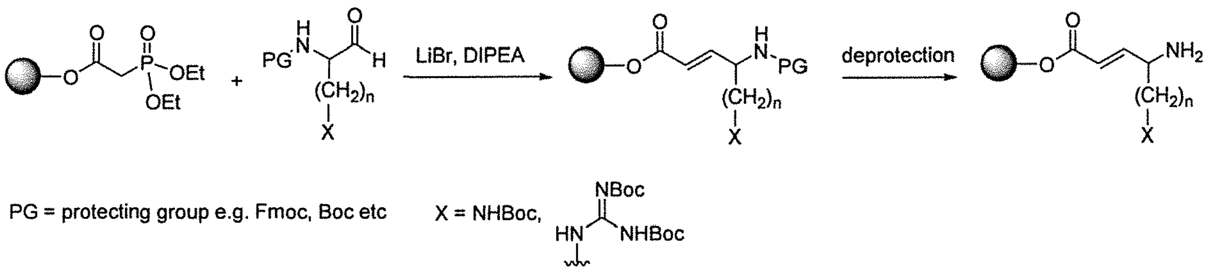


The amount of reagents was calculated estimating a resin loading of 0.50 mmol/g. The resin (300mg, 0.50 mmol*g$^{-1}$, 0.15 mmol, 1.0 eq) was activated by shaking in dry THF (4 mL) for 1 h. A solution of LiBr (26.1 mg, 0.30 mmol, 2.0 eq) and DIPEA (41.1 μL, 0.23 mmol, 1.5 eq), solubilized in dry THF (1 mL), was poured on the resin, followed by the addition of the aldehyde **(Table 2),** also solubilized in dry THF (1 mL). The resin was shaken for 24 h at r.t.

**Table 2.** Amount of α-amino aldehydes.

| Aldehyde | Weight [mg] | n [mmole] | eq |
|---|---|---|---|
| Fmoc-L-Arg(ω,ω'-Boc)-H **(H)** | 261.3 | 0.45 | 3.0 |
| Fmoc-L-Orn(δ-Boc)-H **(I)** | 197.3 | 0.45 | 3.0 |
| Fmoc-L-Lys(ε-Boc)-H **(J)** | 206.8 | 0.46 | 3.1 |

Upon completed reaction, the resin was alternately washed three times with THF (2 mL) and IPA (2 mL).To determine the conversion during HWE reaction, the N-terminus was deprotected by shaking the resin three times with a 20% Piperidine DMF solution (1 mL) for 3 min. Piperidine was removed from the resin by washing alternately the resin three times with DMF (2 mL) and IPA (2 mL), and lastly with DMF (2 mL). An analytical amount of resin was then taken and analyzed using Kaiser test. In case of a positive Kaiser test, the peptide coupling step was perfomed (see **GPF).**

3.2.3. General procedure for peptide coupling **(GPF)**

**The general procedure F** for 300 mg resin, with an estimated loading of 0.50 mmol/g, is summed up in
Table 3. All steps were carried out at room temperature. The coupling step was monitored using the Kaiser test (an analytical amount of the resin was taken, washed with DMF and analysed).

**Table 3.** Procedure for solid phase peptide synthesis.

| Step | Operation | Reagents/Solvent | Volume [mL] | Repeats x time [min] |
|---|---|---|---|---|
| 1 | Coupling | Fmoc-Tyr(*t*Bu)-OH, or fatty acid (0.45 mmol, 3.0 eq), HOBt (50.7 mg, 0.38 mmol, 2.5 eq), HBTU (142.2 mg, 0.38 mmol, 2.5 eq), DIPEA (137.0 μL, 0.75 mmol, 5.0 eq) in DMF | 3 | 1 x 30 |
| 2 | Washing | Alternating, DMF and IPA | 2 | 3 x 1 |
| 3 | Deprotection | 20% piperidine in DMF | 1 | 3 x 3 |
| 4[b] | Washing | Alternating, DMF and IPA | 2 | 3 x 1 |

3.2.4. Cleavage of protected product from resin

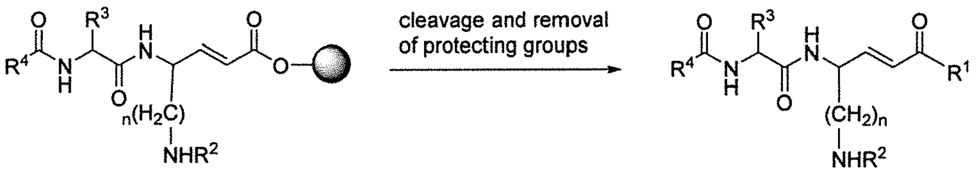


*Wang Resin*: The resin was shaken in 95% TFA, 2.5% TES and 2.5% $H_2O$ (1 mL pro 100 mg resin) for 24 h at r.t. in order to get the unprotected peptide.
*CTT Resin*: The resin was shaken in HFIP:DCM (1:4) (1 mL pro 100 mg resin) for 15 min at r.t. in order to get the protected peptide.
Depending on the quantity of crude product, the peptide was either purified by semi-preparative HPLC (see methods above) or by reverse-phase flash chromatography.

3.2.5. Products

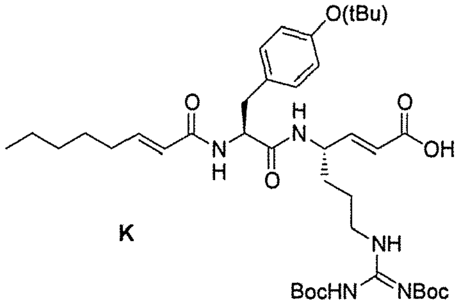

K

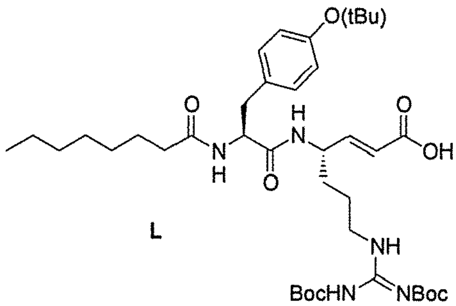

L

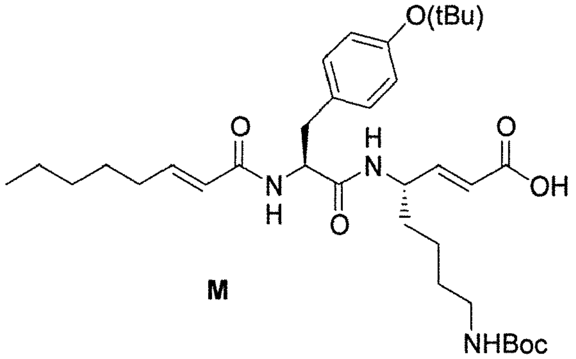

M

Protected barnesin **(K): HRMS (ESI-TOF):** calculated for $C_{39}H_{62}O_9N_5$ $[M+H]^+$ 744.4542; found 744.4537.
Protected 17,18-Dihydrobarnesin **(L): HRMS (ESI-TOF):** calculated for $C_{39}H_{64}O_9N_5$ $[M+H]^+$ 746.4699; found 744.4701.
Protected Lysin-Barnesin **(M): HRMS (ESI-TOF):** calculated for $C_{34}H_{54}O_7N_3$ $[M+H]^+$ 616.3956; found 616.3955.

### *3.3 Modification of protected lipodipeptides*

3.3.1 Synthesis of protected barnesin Weinreb amide (T)

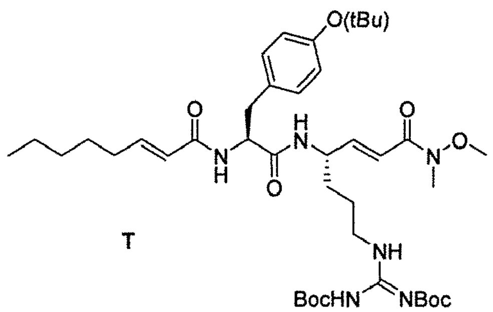

T

Protected barnesin **(K,** 4.5 mg, 6.0 μmol, 1.0 eq) was solubilized in DCM (2 mL) and cooled to 0 °C and then treated with HBTU (1.2 eq) and DiEPA (2.1 eq). Then hydrochloride salt of *N,O*-dimethylhydroxylamine (2.0 eq.) was added. The progress of the reaction was monitored by TLC. The reaction was quenched by aqueous work-up (brine, sodium carbonate solution) and the organic layer was dried over $Na_2SO_4$ and evaporated under reduced pressure. The pure Boc-Weinreb amide was isolated after column chromatography as colourless oil.
Protected barnesin weinreb amide **(T): HRMS (ESI-TOF):** calculated for $C_{41}H_{67}N_6O_9$ $[M+H]^+$ 787.4970; found 787.4969.

3.3.2 Modifications of C-terminus (GPG)

The protected purified peptide was solubilized in dry MeOH (2 mL) and cooled to 0 °C. TMS-diazomethane (5.0 eq) was added dropwise. A production of gas ($N_2$) was observed and the solution turned yellow. The reaction mixture

was stirred at r.t. The reaction was monitored by UHPLC-MS and in case of uncomplete conversion additional TMS-diazomethane (5.0 eq) was added every hour until full conversion of the peptide into the methyl ester. The reaction mixture was concentrated to obtain a yellowish oil, which was purified by semi preparative HPLC.

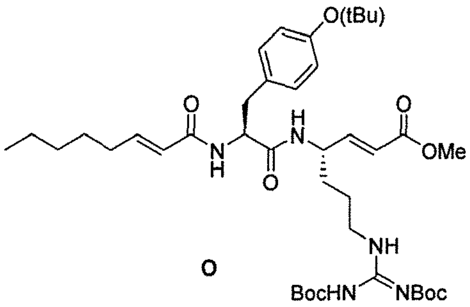

**O**

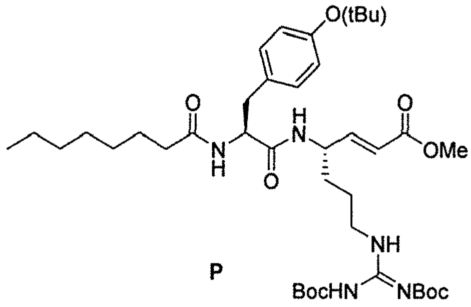

**P**

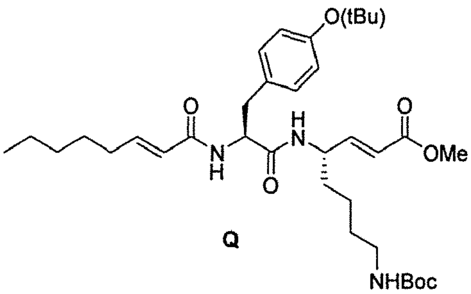

**Q**

Protected barnesin methyl ester (**O**): According to **GPG,** protected peptide **(K,** 4.5 mg, 6.0 μmol, 1.0 eq) was converted to ester (**O**, 2.5 mg after purification, 3.3 μmol, 53% yield, colourless oil). **HRMS (ESI-TOF):** calculated for $C_{40}H_{64}O_9N_5$ $[M+H]^+$ 758.4699; found 758.4694.

Protected 17,18-dehydrobarnesin methyl ester **(P):** According to **GPG,** protected peptide **(L,** 5.6 mg, 7.5 μmol, 1.0 eq) was converted to ester **(P)** (2.7 mg after purification, 3.6 μmol, 47% yield, colourless oil). **HRMS (ESI-TOF):** calculated for $C_{40}H_{66}O_9N_5$ $[M+H]^+$ 760.4855; found 758.4860.

Protected lysin-barnesin methyl ester **(Q):** According to **GPG,** protected peptide **(M,** 6.7 mg, 10.5 μmol, 1.0 eq) was converted to ester **(Q)** (2.6 mg after purification, 4.1 μmol, 38% yield, colourless oil). **HRMS (ESI-TOF):** calculated for $C_{35}H_{56}O_7N_3$ $[M+H]^+$ 630.4113; found 630.4114.

3.3.3 Deprotection ***(GPH)***

The protected purified peptide was cooled to 0°C. A solution of 95 % TFA in DCM (1 mL) was poured on the peptide and the mixture was stirred and allowed to warm to r.t. over 24h.. Upon completed reaction, the mixture was concentrated to obtain the deprotected peptide as yellowish oil. It was purified on semi-preparative HPLC.

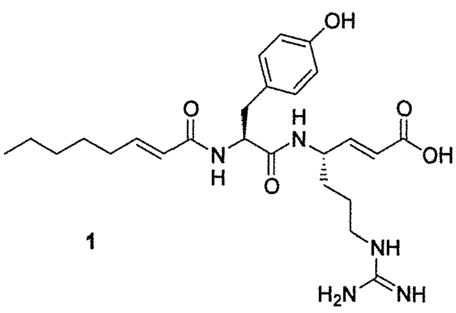

**1**

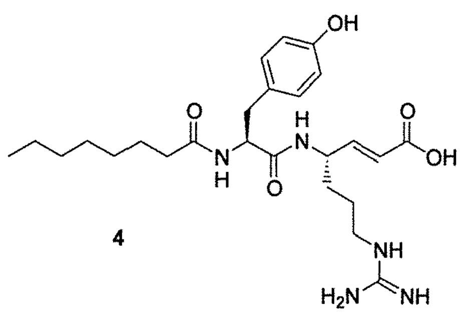

**4**

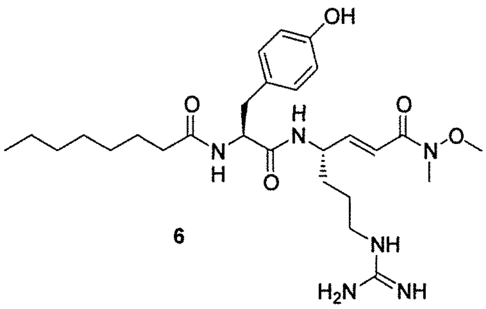

6

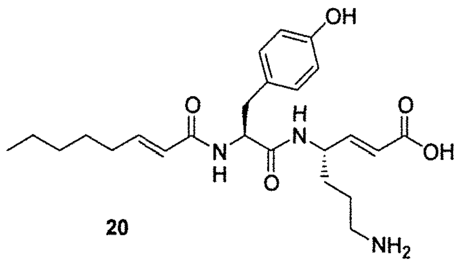

20

Barnesin A **(1):** According to **GPH,** protected barnesin (**O**, 4.4 mg, 5.9 μmol, 1.0 eq) was converted to barnesin **(1)** (1.5 mg, 3.1 μmol, 52% yield, colourless oil). **HRMS (ESI-TOF):** calculated for $C_{25}H_{38}O_5N_5$ $[M+H]^+$ 488.2867; found 488.2871.

17,18-Dihydrobarnesin **(4):** According to **GPH,** protected purified 17,18-dihydrobarnesin **(P,** 5.6 mg, 7.5 μmol, 1.0 eq) was converted to 17,18-dihydrobarnesin **(4,** 3.5 mg, 7.1 μmol, 95% yield, colourless oil). **HRMS (ESI-TOF):** calculated for $C_{25}H_{40}O_5N_5$ $[M+H]^+$ 490.3024; found 490.3020. **IR (ATR)** $\mathbf{v_{max}}$**:** 3273, 2956, 2927, 2856, 1632, 1541, 1516, 1457, 1393. $[\alpha]_D^{25}$: -9.0° (c 1.0; MeOH). Weinreb barnesin **(6):** According to **GPH,** protected purified weinreb barnesin **(T,** 6 mg, 7.6 μmol, 1.0 eq.) was converted to Weinreb barnesin **(6,** 3 mg, 5.6 μmol, 73%, colourless oil). **HRMS (ESI-TOF):** calculated for $C_{27}H_{44}N_6O_5$ $[M+H]^+$ 533.3451; found 533.3453.

Lysin-barnesin **(20):** According to **GPH,** protected purified lysin-barnesin **(Q,** 4.5 mg, 7.3 μmol, 1.0 eq) was converted to lysin-barnesin **(20,** 2.5 mg, 5.4 μmol, 74% yield, colourless oil). **HRMS (ESI-TOF):** calculated for $C_{25}H_{38}O_5N_3$ $[M+H]^+$ 460.2806; found 460.2801.

### *3.4 Preparation of Comparative Compounds R and S*

3.4.1 Synthesis of protected 2,3-17,18-Tetrahydrobarnesin (N)

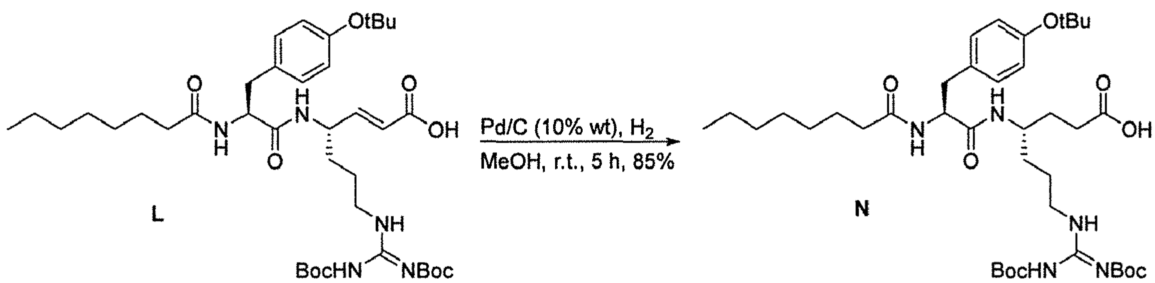


Peptide **(L)** (16 mg, 21.4 μmol, 1.0 eq) was solubilized in methanol and put under argon atmosphere in a dried round-bottom flask. A spatula tip of Pd on carbon (10% weight) was added to the solution. The suspension was put under $H_2$ atmosphere, and stirred at r.t. for 5 h. An analysis by UPLC-MS confirmed the completion of the reaction. The reaction mixture is filtered over Celite and washed with methanol. The solution was then dried under vacuum to obtain hydrogenated peptide **(N)** (13.6 mg, 18 μmol, 85% yield) as yellowish oil. **HRMS (ESI-TOF):** calculated for $C_{39}H_{66}O_9N_5$ $[M+H]^+$ 747.4855; found 748.4847.

3.4.2 Preparation of Comparative Compound **R**

2,3,17,18-Tetrahydrobarnesin A **(R):** According to **GPH** (see section 3.3.3 above) protected purified 2,3-17,18-tetrahydrobarnesin **(N,** 12 mg, 16.0 μmol, 1.0 eq) was converted to 2,3-17,18-tetrahydrobarnesin **(R,** 3.8 mg, 7.7 μmol, 48% yield, colourless oil).

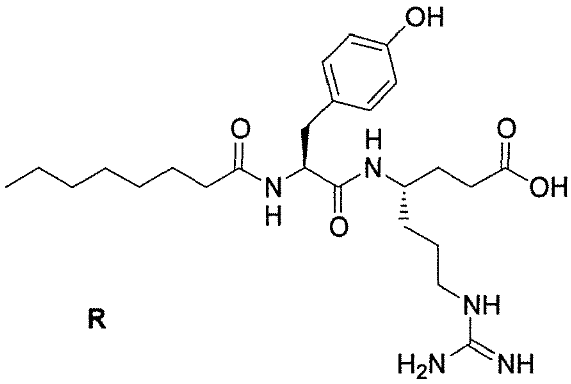

**R**

**HRMS (ESI-TOF):** calculated for $C_{23}H_{42}O_5N_5$ $[M+H]^+$ 492.3180; found 492.3177. **IR (ATR) $v_{max}$:** 3288, 2949, 2838, 1644, 1557, 1518, 1449, 1402. $[\alpha]_D^{25}$: +8.3° (c 1.0; MeOH).

3.4.3 Preparation of Comparative Compound **S**

2,3-17,18-Tetrahydrobarnesin A methyl ester **(S):** Treatment of **R** with acidic methanol afforded methyl ester (**S**, 2.9 mg, 5.7 μmol, colourless oil).

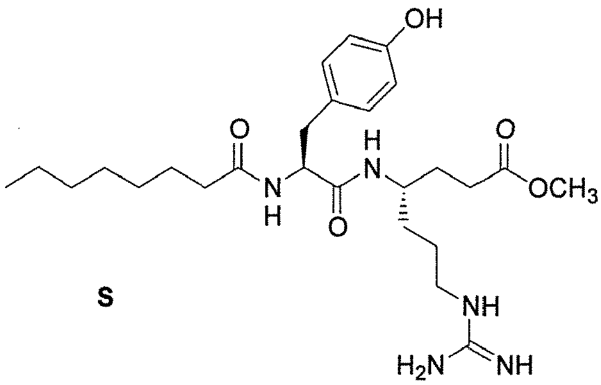

**S**

**HRMS (ESI-TOF):** calculated for $C_{26}H_{44}O_5N_5$ $[M+H]^+$ 506.3337; found 506.3336. **IR (ATR) $v_{max}$:** 3280, 2929, 2856, 1652, 1540, 1517, 1438. $[\alpha]_D^{25}$: +10.22° (c 1.0; MeOH).

**Example 4 Assessment of protease inhibition**

**Protease Assays with azocasein:** Protease inhibition assays against the proteases papain, ficin, trypsin, pepsin and thermolysin were performed according to the protocol of García-Carreño (loc. cit.) in a reaction buffer containing 25 mM TRIS-HC1, 0.15 M NaCl, pH 7.2 (or pH 3.5 for aspartic protease). The total assay volume was 47 μL buffer with 2 μL of the protease as well as 1 μL of suitable inhibitor or corresponding inhibitor solvent (100 % MeoH or water). Final concentration of proteases was as follows: 2 mg/mL for papain (Sigma-Aldrich, P4762), 600 μg/mL for ficin (Sigma-Aldrich, F6008), 80 μg/mL for trypsin (Thermo Fisher, 23266), 60 mg/mL for pepsin (Sigma-Aldrich P6887)) and 2 μg/mL for thermolysin (Sigma-Aldrich, P1512).

Inhibitors were dissolved in 100% MeOH or water and added to the assay (1 μL); final concentration: 2% MeOH. Samples of control inhibitors were prepared as follows: (1) serine protease (trypsin): PMFS (2 mM) and soybean trypsin inhibitor (240 μM) (Sigma-Aldrich, T1021), (2) cysteine proteases (papain und ficin): iodacetamide (2 mM), (3) asparagine protease (pepsin): pepstatin A (1 μg/mL) (Sigma-Aldrich, P4265), (4) metalloprotease (thermolysin): EDTA (10 mM). In all cases the given concentration inhibited enzyme activity > 95 %. Negative controls were performed with protease alone (without inhibitor) and MeOH at a final concentration of 2 % (reference for activity of 100 %). Extract absorbance controls were performed using inhibitor (1 μL in MeOH) without protease. Blanks were performed using 49 μL buffer

and 1 μL MeOH.

Samples were incubated with buffer and suitable inhibitor (or without for negative controls) for 1 h at r.t. and the reaction was started afterward by adding 50 μL of 1 % azocasein substrate and incubated for 1 h at 37 °C. After stopping the reaction with trichloracetic acid (TCA), the separation of the precipitate was accomplished by centrifugation at 6500 g for 5 min. The hydrolyzed substrate supernatant was incubated in an equal volume of 0.5 N NaOH for 5 min and the absorbance was measured at 440 nm. OD values of the protease control without inhibitor were used as reference for a 100 % activity.

The log $IC_{50}$ values were determined using the standard curves analyzing tool with four parameter logistic equation of SigmaPlot12 with technical triplicates. Propagation of error was calculated using the standard error and log $IC_{50}$ values where the equation of error propagation is defined as $\Delta y = 0.43\Delta x/x$.

**Cathepsin B inhibition assay.** Cathepsin B inhibition assay was determined according to Hiwasa et al. (loc. cit.) with minor changes. Cathepsin B was purchased from Sigma Aldrich (C0150) and stored in 50 mM sodium acetate, 1 mM EDTA, pH 5 (adjusted with acetic acid). Cathepsin B was activated by preincubation at 40 °C for 10 min in assay buffer (0,1 M sodium acetate 1.3 mM EDTA, pH 6.0 adjusted with acetic acid, 2 μM DTT, 2,6 mM cysteine and 0,05 % Triton X100). The total assay volume was 47 μL buffer with 2 μL cathepsin B as well as 1 μL of suitable inhibitor or corresponding inhibitor solvent (100 % MeOH or water). Final concentration of cathepsin B was 0,1 mg/ml. Samples were incubated with buffer and suitable inhibitor (or without for negative controls) for 20 min at 4 °C and the reaction was started afterwards by adding 1 μL of 10 mM Z-Arg-Arg-AMC **(Peptanova,** 3123-v) and incubated for 20 min at 40 °C. After stopping the reaction with 85 μL buffer containing 100 mM sodium monochlor acetate, 30 mM sodium acetate, 70 mM acetic acid, the hydrolyzed substrate was detected at an excitation wavelength of 380 nm and a fluorescence wavelength of 450 nm, using a fluorophotometer. 1 μL of 1.5 mM leupeptin was used as enzyme inhibition control.

The results of the inhibition assays are summarized in Tables 4A and B below.

***Table 4A: Evaluation of protease inhibition activities***

| **Compound** | **Protease class** (inhibitory activity as IC50 value (μM)) | | | | | |
|---|---|---|---|---|---|---|
| | **Cysteine (3.4.22)** | | | **Serine (3.4.21)** | **Aspartic (3.4.23)** | **Metallo (3.4.24)** |
| | **Papain** | **Ficin** | **Cathepsin B** | **Trypsin** | **Pepsin** | **Thermolysin** |
| **Barnesin A (1)** | 15.96 μM (± 5.8) | 3.43 μM (± 0.15) | 91.72 nM (± 5.8) | n.i. | n.i. | n.i. |
| **Barnesin A ethyl ester (3)** | 2.89 μM (± 0.13) | 3.43 μM (± 0.38) | 23.99 nM (± 0.13) | n.i. | n.i. | n.i. |
| **17,18-Dihydrobarnesin (4)** | 4.78 μM (± 2.9) | 1.16 μM (± 0.07) | 87.56 nM (± 2.9) | n.i. | n.i. | n.i. |
| | | | | | | |
| **Comp. Compound (R)** | n.i. | n.i. | n.i. | n.i. | n.i. | n.i. |
| **Comp. Compound (S)** | n.i. | n.i. | n.i. | n.i. | n.i. | n.i. |

***Table 4B: Reported protease inhibition activities of known compounds***

| **Compound** | **Protease class** (inhibitory activity as IC50 value (μM)) | | | | | |
|---|---|---|---|---|---|---|
| | **Cysteine (3.4.22)** | | | **Serine (3.4.21)** | **Aspartic (3.4.23)** | **Metallo (3.4.24)** |
| | **Papain** | **Ficin** | **Cathepsin B** | **Trypsin** | **Pepsin** | **Thermolysin** |
| Leupeptin | 0.86 μM | n.r. | 21.5 nM | 2.2 μM | n.r. | n.r. |
| Cyclopropenone 1'S | 0.0054 μM | n.r. | 0.71 μM | n.r. | n.r. | n.r. |
| Cyclopropenone 1'R | 22 μM | n.r. | 0.044 μM | n.r. | n.r. | n.r. |

(continued)

| Compound | Protease class (inhibitory activity as IC50 value (μM)) | | | | | |
|---|---|---|---|---|---|---|
| | Cysteine (3.4.22) | | | Serine (3.4.21) | Aspartic (3.4.23) | Metallo (3.4.24) |
| | Papain | Ficin | Cathepsin B | Trypsin | Pepsin | Thermolysin |
| Cystatin | 0.029 μM | n.r. | n.r. | 3.5 μM | n.r. | n.r. |
| Miraziridine A | n.r. | n.r. | 2.05 μM | 60 μM | n.r. | n.r. |
| Tokaramide A | n.r. | n.r. | 62.4 nM | n.r. | n.r. | n.r. |
| YM 51084 | 2.2 μM | n.r. | 12.0 nM | n.r. | n.r. | n.r. |
| a) n.r. = not reported in literature; n.i. = no inhibition | | | | | | |

The above results confirm that the compounds according to the invention act as selective cysteine protease inhibitors in the low molecular range.

The features of the present invention disclosed in the specification, the claims and/or the drawings may both separately and in any combination thereof be material for realizing the invention.

## Claims

1. A compound of the general formula (I):

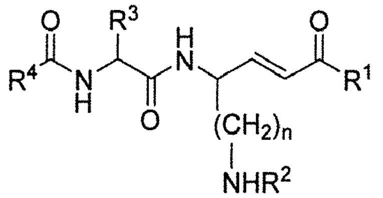

(I)

or a pharmacologically acceptable salt thereof, wherein

$R^1$ represents a hydrogen atom, $-OR^{11}$, $-NR^{11}R^{12}$; or a $(C_1\text{-}C_6)$alkyl, $(C_2\text{-}C_6)$alkenyl, $(C_2\text{-}C_6)$ alkinyl, or $(C_3\text{-}C_6)$ cycloalkyl group, all of which groups may optionally be substituted;
$R^{11}$ and $R^{12}$ each, independently of one another, represents a hydrogen atom, or a $(C_1\text{-}C_6)$alkyl, $(C_2\text{-}C_6)$alkenyl, $(C_2\text{-}C_6)$ alkinyl or $(C_1\text{-}C_6)$heteroalkyl group, all of which groups may optionally be substituted;
$R^2$ is a hydrogen atom, a group of formula $-C(=NH)NH_2$, or a group of formula $-C(=O)R^{21}$;
$R^{21}$ represents a $(C_1\text{-}C_6)$alkyl, $(C_2\text{-}C_6)$alkenyl, $(C_2\text{-}C_6)$ alkinyl or $(C_1\text{-}C_6)$heteroalkyl group; all of which groups may optionally be substituted;
$R^3$ is an amino acid side chain; a hydrogen atom, a halogen atom, OH; or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group; all of which groups may optionally be substituted;
$R^4$ is an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group; all of which groups may optionally be substituted; and
n is an integer of from 1 to 6.

2. The compound according to claim 1, or a pharmacologically acceptable salt thereof, wherein $R^1$ is a hydrogen atom, -OH or a $C_{1\text{-}3}$ alkoxy group.

3. The compound according to claim 1 or 2, or a pharmacologically acceptable salt thereof, wherein $R^2$ is a hydrogen atom, a group of formula $-C(=NH)NH_2$, a group of formula $-C(=O)CH_3$, or a group of formula $-C(=O)CH_2CH_2CH_3$.

4. The compound according to any one of claims 1 to 3, or a pharmacologically acceptable salt thereof, wherein $R^3$ is an amino acid side chain of a proteinogenic amino acid; or a group of formula (II):

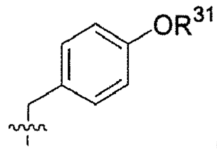

(II),

wherein $R^{31}$ represents a $(C_1\text{-}C_6)$alkyl, $(C_2\text{-}C_6)$alkenyl, or $(C_2\text{-}C_6)$ alkinyl group, all of which groups may optionally be substituted. Preferably, $R^{31}$ represents an optionally substituted $(C_1\text{-}C_4)$alkyl, $(C_2\text{-}C_4)$alkenyl, or $(C_2\text{-}C_4)$ alkinyl group; and more preferably $R^{31}$ is selected from methyl, prop-2-enyl, prop-2-inyl, but-3-enyl and but-3-inyl.

5. The compound according to any one of claims 1 to 4, or a pharmacologically acceptable salt thereof, wherein $R^4$ is a $C_{1\text{-}7}$ alkyl or $C_{2\text{-}7}$ alkenyl group; which groups may optionally be substituted.

6. The compound according to any one of claims 1 to 5, or a pharmacologically acceptable salt thereof, wherein n is 3 or 4.

7. The compound according to any one of claims 1 to 6, wherein the compound is:

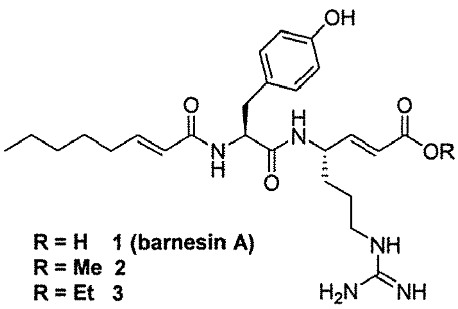

**R = H 1 (barnesin A)**
**R = Me 2**
**R = Et 3**

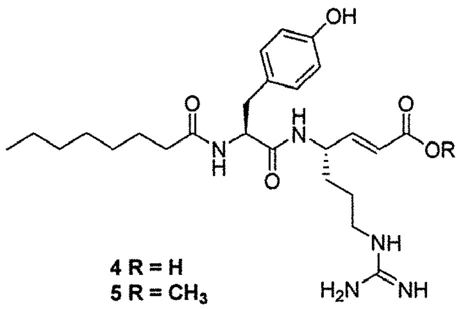

**4 R = H**
**5 R = $CH_3$**

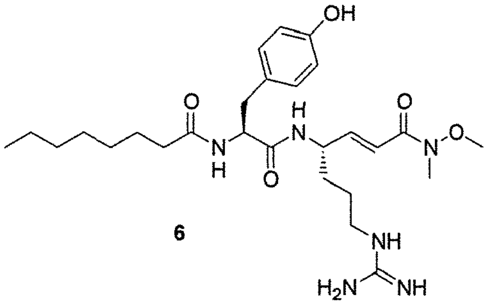

**6**

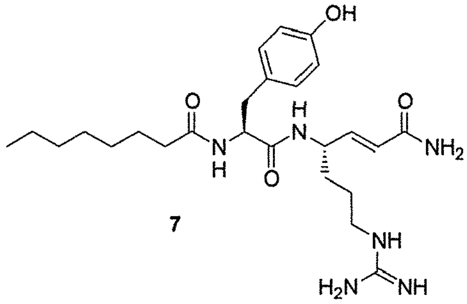

**7**

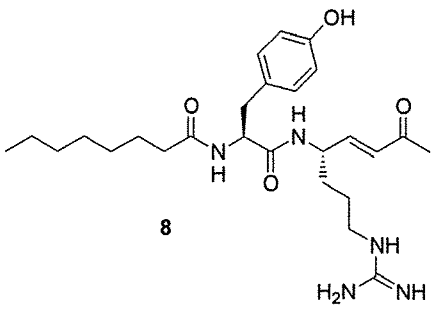

**8**

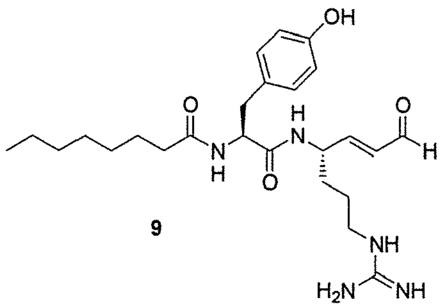

**9**

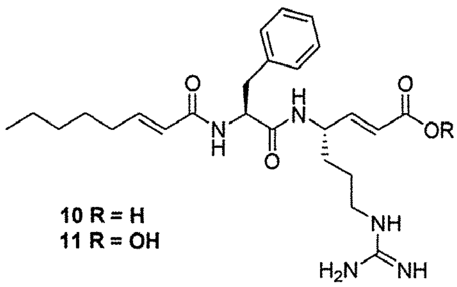

**10 R = H**
**11 R = OH**

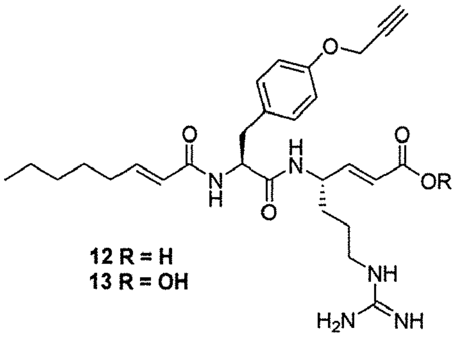

**12 R = H**
**13 R = OH**

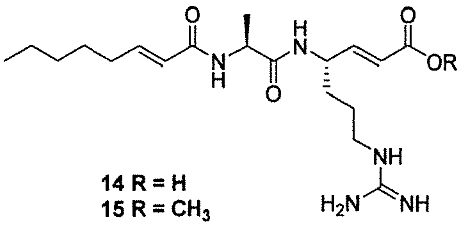

**14 R = H**
**15 R = $CH_3$**

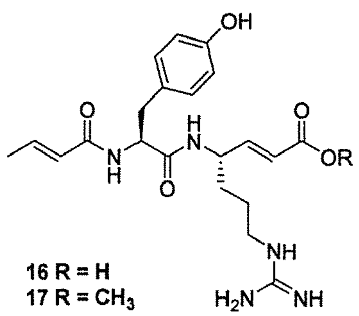

**16 R = H**
**17 R = $CH_3$**

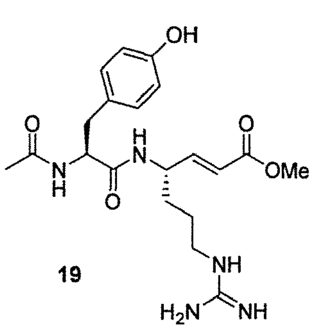

**19**

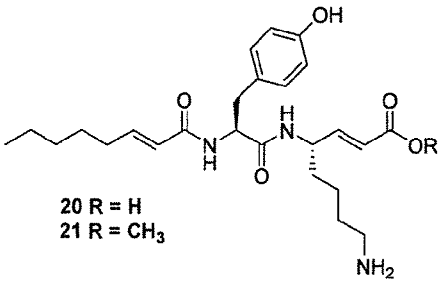

**20** R = H
**21** R = $CH_3$

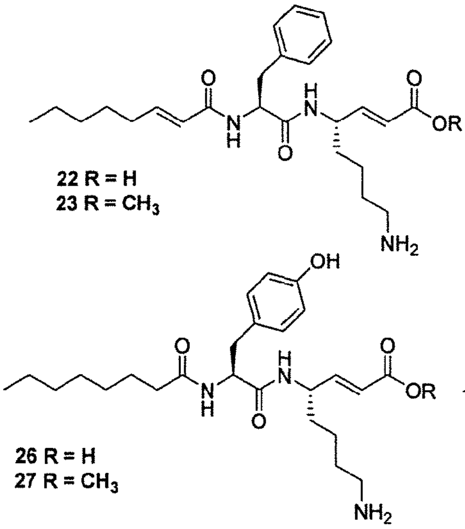

**22** R = H
**23** R = $CH_3$

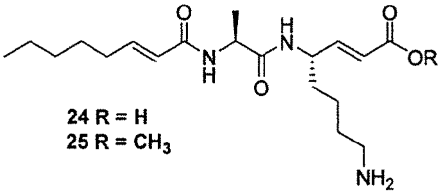

**24** R = H
**25** R = $CH_3$

**26** R = H
**27** R = $CH_3$

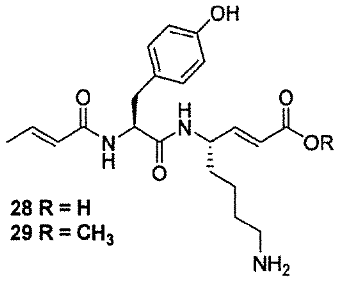

**28** R = H
**29** R = $CH_3$

or a pharmacologically acceptable salt thereof.

**8.** A pharmaceutical composition comprising at least one compound according to any one of claims 1 to 7 and, optionally, one or more carrier substance(s), excipient(s) and/or adjuvant(s).

**9.** A combination preparation containing at least one compound according to any one of claims 1 to 7 and at least one further active pharmaceutical ingredient.

**10.** The compound according to any one of claims 1 to 7, the pharmaceutical composition of claim 8, or the combination preparation of claim 9 for use as a medicament.

**11.** The compound according to any one of claims 1 to 7, the pharmaceutical composition of claim 8, or the combination preparation of claim 9 for use in the prevention and/or treatment of a condition or disorder associated with a pathophysiological level of a proteasome or a cysteine protease.

**12.** The compound for use according to claim 11, wherein the condition or disorder associated with a pathophysiological level of a proteasome or a cysteine protease is a neurodegenerative disorder, a parasitic infection, an invasive cancer, or a metastatic cancer.

**13.** The compound according to any one of claims 1 to 7, or the pharmaceutical salt thereof, for use as an inhibitor of a proteasome or a cysteine protease.

**14.** A synthetic nucleic acid comprising a sequence encoding a nonribosomal peptide-synthetase (NRPS) - polyketide synthase (PKS) gene cluster, wherein the sequence has a sequence identity to the full-length sequence of SEQ ID NO. 1 from at least 90%, 95%, 96%, 97%, 98%, 98.5%, 99%, or 99.5% to 100%.

**15.** A method for the preparation of compound **(1)** of claim 7, the method comprising the steps of:

(a) fermenting *Sulfurospirillum barnesii* (DSM 10660); and
(b) separating and retaining the compound according to general formula (I) from the culture broth.

Europäisches Patentamt
European Patent Office
Office européen des brevets

# EUROPEAN SEARCH REPORT

Application Number
EP 18 16 0274

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ISMAIL F M D ET AL: "Aziridine alkaloids as potential therapeutic agents", EUROPEAN JOURNAL OF MEDICINAL CHEMISTRY, EDITIONS SCIENTIFIQUE ELSEVIER, PARIS, FR, vol. 44, no. 9, 1 September 2009 (2009-09-01), pages 3373-3387, XP026322159, ISSN: 0223-5234, DOI: 10.1016/J.EJMECH.2009.05.013 [retrieved on 2009-05-22] | 1-3,5,6, 8-13 | INV. C07C279/14 A61P33/00 C07H21/00 C12P13/02 A61P25/28 A61P35/04 A61P35/00 A61K31/155 |
| A | * page 3378; table 3 * * page 3379, left-hand column * ----- | 4,7 | |
| X | SCHASCHKE NORBERT: "Miraziridine A: natures blueprint towards protease class-spanning inhibitors", BIOORGANIC & MEDICINAL CHEMISTRY LETTERS, vol. 14, no. 4, 2004, pages 855-857, XP085048697, ISSN: 0960-894X, DOI: 10.1016/J.BMCL.2003.12.030 | 1-3,5,6, 8-13 | |
| A | * scheme 3; compound 15 * * table 1 * ----- | 4,7 | TECHNICAL FIELDS SEARCHED (IPC) C07C A61P C07H C12P |
| X | KONNO ET AL: "Total synthesis of miraziridine A and identification of its major reaction site for cathepsin B", TETRAHEDRON, ELSEVIER SCIENCE PUBLISHERS, AMSTERDAM, NL, vol. 63, no. 38, 8 August 2007 (2007-08-08), pages 9502-9513, XP022190282, ISSN: 0040-4020, DOI: 10.1016/J.TET.2007.06.082 | 1-3,5,6, 8-13 | |
| A | * page 9503; compounds 1,3 * * page 9506; table 3 * ----- | 4,7 | |

~~The present search report has been drawn up for all claims~~

2

| Place of search | Date of completion of the search | Examiner |
|---|---|---|
| Munich | 17 May 2018 | Österle, Carmen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 18 16 0274

## CLAIMS INCURRING FEES

The present European patent application comprised at the time of filing claims for which payment was due.

- [ ] Only part of the claims have been paid within the prescribed time limit. The present European search report has been drawn up for those claims for which no payment was due and for those claims for which claims fees have been paid, namely claim(s):

- [ ] No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

- [ ] All further search fees have been paid within the fixed time limit. The present European search report has been drawn up for all claims.

- [ ] As all searchable claims could be searched without effort justifying an additional fee, the Search Division did not invite payment of any additional fee.

- [ ] Only part of the further search fees have been paid within the fixed time limit. The present European search report has been drawn up for those parts of the European patent application which relate to the inventions in respect of which search fees have been paid, namely claims:

- [X] None of the further search fees have been paid within the fixed time limit. The present European search report has been drawn up for those parts of the European patent application which relate to the invention first mentioned in the claims, namely claims:

  1-13(partially)

- [ ] The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 18 16 0274

The Search Division considers that the present European patent application does not comply with the requirements of unity of invention and relates to several inventions or groups of inventions, namely:

1. claims: 1-13(partially)

   Compounds of general formula (I) for which R4 is defined as alkyl, pharmaceutical comprising such compounds and the medical use of these compounds

   ---

2. claims: 15(completely); 1-13(partially)

   Compounds of general formula (I) for which R4 is defined as alkenyl, pharmaceutical comprising such compounds and the medical use of these compounds;
   Process for preparing compound (1) of claim 7

   ---

3. claims: 1-4, 6, 8-13(all partially)

   Compounds of general formula (I) for which R4 is defined as alkynyl, pharmaceutical comprising such compounds and the medical use of these compounds

   ---

4. claims: 1-4, 6, 8-13(all partially)

   Compounds of general formula (I) for which R4 is defined as heteroalkyl, pharmaceutical comprising such compounds and the medical use of these compounds

   ---

5. claims: 1-4, 6, 8-13(all partially)

   Compounds of general formula (I) for which R4 is defined as cycloalkyl, heterocycloalkyl, alkylcycloalkyl and heteroalkylcycloalkyl, pharmaceutical comprising such compounds and the medical use of these compounds

   ---

6. claims: 1-4, 6, 8-13(all partially)

   Compounds of general formula (I) for which R4 is defined as aryl, heteroaryl, aralkyl, heteroaralkyl, pharmaceutical comprising such compounds and the medical use of these compounds

   ---

7. claim: 14

   Synthetic nucleic acid comprising a sequence with a sequence identity of at least 90% to the full length sequence of SEQ ID NO. 1.

   ---

Europäisches Patentamt
European Patent Office
Office européen des brevets

## LACK OF UNITY OF INVENTION SHEET B

Application Number
EP 18 16 0274

The Search Division considers that the present European patent application does not comply with the requirements of unity of invention and relates to several inventions or groups of inventions, namely:

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ZIEMERT ; JENSEN.** *Methods in enzymology,* 2012, vol. 517, 161
- **GORIS ; DIECKERT.** The genus Sulfurospirillum. In Organohalide-respiring bacteria. Berlin Heidelberg: Springer, 2016
- **GORIS et al.** *Environmental microbiology,* 2014, vol. 16 (11), 3562-3580
- **SIKLOS et al.** *Acta Pharmaceutica Sinica B,* 2015, vol. 5 (6), 506-519
- **FERREIRA ; ANDRICOPULO.** *Pharmacology & Therapeutics,* 2017, vol. 180, 49-61
- **MASON ; JOYCE.** *Trends in Cell Biology,* 2011, vol. 21 (4), 228-237
- **GARCÍA-CARREÑO.** *Biotechnology Education,* 1992, vol. 3, 145-150
- **HIWASA et al.** *Cancer letters,* 1993, vol. 69, 161-165
- **MICHAEL B. SMITH ; JERRY MARCH.** March's Advanced Organic Chemistry: Reactions, Mechanisms, and Structure. John Wiley & Sons, 2007

SEQUENCE LISTING

<110> Leibniz Institute for Natural Product Research and Infection Biology e.V. - Hans-Knöll-Institut (HKI)

<120> Barnesin A, derivatives and uses thereof

<130> 22438-EP

<160> 1

<170> PatentIn version 3.5

<210> 1
<211> 16035
<212> DNA
<213> Artificial Sequence

<220>
<223> Barnesin A expression cassette

<220>
<221> misc_feature
<222> (1)..(16035)
<223> Barnesin A expression cassette

<400> 1
ccatcgaatg gccagatgat taattcctaa tttttgttga cactctatca ttgatagagt 60

tattttacca ctccctatca gtgatagaga aaagtgaaat gaatagttcg acaaaaatct 120

agaaataatt ttgtttaact ttaagaagga gatataccat ggatgcttaa tatatacgca 180

ctttcatcac cacaagaggc tatatatatt gactcccttt tgtatagtga aaccacaaaa 240

tatactatgg gtggctttgc tctcttaagt ggtcggattg atgttgagat atttaaaaaa 300

gcccatgcca ttacactgac ccttcacgat gtatttcttt ccaaaataga gacaaggcat 360

gggaaaattg tacaaagctc tccacatgaa ccatatgggg tatattttaa ggatttttcc 420

acttccgttg attcatacag ggcaggtgtt gagttcattc ttgaagattt ttctcatccc 480

atacccatca atgcataccc ccttgcagca gatatgctac tcaaagtgaa tgaagagctt 540

tatatttggt acaccaaact gcatcatatt atgaacgacg catttggtca tgctcttttt 600

gcagaaactt tatctgaaat ttatacagct ttgttttata agcaagagtt gccagtaaaa 660

gaaaggcaca gatacgttga ttttgttgaa gatgacaacg catatctcca ttccgaaacc 720

tttttaaaag atgcagcatt ttggatggag aagtttaaga ctctacctga acctatttct 780

ttttcaggtt caaaagatgg actttgtgag caagaagcat taaaaacgga aagaatgaca 840

ttaacactta gcagattgtg ttacaacgat atgctcagga tttgtagcga aaatagtttt 900

acctcctttc attttcttct tgccgttctt tatacgtatt tgtatagaac aaaaaaccgc 960

aacgatattg ttattggcat gcccattctt aatcgcaata ataaaaaatt tagaaacact 1020

tcgggtatgt ttatgggtat gataccccctt cgtattaaaa tggaagaggg tatgagtttt 1080

atggatctag cgcttggtat aagccgtgaa cttagggagt gttatcggca tcagcgttac 1140

cctcttggtc agataataaa ggattgccgt agcaaagagg ggttttatgg taacattttt 1200

gatataacat ttgtgtaccg taaactttcc tatgataaga agtttggaaa aactcttatg 1260

cgcatgcaaa tacttgatac aaaagcgagg gaagaatcct tttccgttga agtagatgag 1320

tttgacgatg gcgatgtgaa tcttttttt aattacaatc cgtatgttat tcctccagaa 1380

gaggcagaac agatggttag agggtttgaa accctttttt tagatgttgc aatgatggaa 1440

gataagcctc ttaaagagct tagaattatg cctgaaagag tgaggtgtgg ggtttttgga 1500

gaaaaaatta actttcctaa aaagcgattt gaagaaatct ttgcacagac atgtatgcag 1560

tatggcgcta aagaagccgt aaaatacaaa gacactactc ttacatacgt tgaattagat 1620

ggcttctctt caggcatcgc caaaaaacta agagacgatt ttgggctaca aaaaggtgac 1680

agggttgttt gtgttcttga gcgtactcac attgtgccag tggtgatact tgcccttttt 1740

aaaatggggt gtgtgtatgt tccagccgat acgtcccttc ctaaagatcg tatcaattac 1800

atggtaaatg attgtgcagc aaagctaatt atcagttcag ctcctcttga agcagaggtt 1860

tgcgtctata tacccgatac tctagtttca ggcttttttg cacccataga gagtttaaat 1920

gctgaagatg atgcttatat catatacact tctggaacaa caggtcgtcc taaaggggtt 1980

gttgtaccgc atcaaggaat agtcaatacg gtgttgtcac aagcaagtca atgggagggt 2040

ggtaagcatg ccaatgttct ccaatttgct tctttaggat ttgatgcctc cttgtcagag 2100

attgggatgg cgctccttag tggcgcatcc ttaaagatcg tgccaaagca gacaattcta 2160

agtcctgctg agtttataga ttttatgaac aaagaaaagg ttacagtagc aacattgcct 2220

ccttcttatc tgagttcatt agggttccct gaattttttt atcttaaaag cttaataacc 2280

gcaggtgaat cacccataga aaaagatgtt ctttttttaca aaaacctctg tcgtgtcatc 2340

aacgcatacg gacccacaga aatatcagtg tgcgcaagtt ggtacgaaat ccccaaagac 2400

tattgtgggg gcgctgttcc cataggaaaa gcaattgata atgcacatat ttatattttg 2460

gatgataacc tcaacccccct tccgcttggt tctgtggggc agatatgtgt aggaggagca 2520

ggtgttacta ggggatacct taataatcca gaactaacgc agcaaacatt tcaaaaagac 2580

ctcttttttg aggggcagaa aatgtatcta acgggggata tggggaaaat taatgctgat 2640

ggtaaccttg tgtttttagg aagaaaagat gcacagatta agcttagggg ttatcgcatt 2700

gagccagaag aggtagcacg tgtgatggag ggctttctt ttatagatgc cacatccatt 2760

gatgttcacg acgaaggtga aagcaaaagc cttgtagcgt attacactgc aaaaaatgta 2820

gttgatgttg cgtatattaa aaaggcactt ttggaaaaat tacctcctta tatgattccc 2880

tcattttta tacatgtaaa agaatttcct cttaatgtta gtgggaaaat agacaaaaaa 2940

gcactcaaag gggcatttaa aaaaatacct tcttctacaa aagagctcca acttccgcta 3000
agtgaaacag agcagaatgt ttctcgtgtg tggtcagaaa ttcttgagat agatcacttt 3060
gaaacggata caaacttttt tgatttaggt gggcattctt tgcatgcgat taaggtcatg 3120
agcaaactgt tcactctctt tggtgtgcgc attgggttaa gggagttttt tgcagaccca 3180
acgcttaagg gtattgcttc tattctggac aaaaggagcg gcaataaaga gaaaaaaatc 3240
gcaaatcttg cattgccaca taccagagcg ttaagtccat cggaaagacg tatttgggtg 3300
ttttgcgcaa tgcaaggtag ctcttctgct tataatatgc ctcttctcat ggacattgaa 3360
ggagaggtgg atgtaggtgc gctaaacagt gctcttaagc ttattctctc acggcatgat 3420
attttaagaa cctattatga agaagaaagg gctgtgtgcc acatcaaaag tgagtgcatg 3480
tttgaagttc aagaaatcgt caccaaaaac ctcactgagg cactcttgca tgagattgat 3540
gaacctttca atttatacga tgcgcccctt ttcaggttga agatattgac tgatggaacc 3600
aaaaaaacat tgagcttcgt gattcatcac atcatttcag acggttggtc gcttcaagtg 3660
ttgatgaacg aattgcttca agcctacgaa gcggtgaaaa aaagagaagt gcccgagctt 3720
tcaccaacaa atgtttctta cgcctcttat gccacatggt tagagagtga agagtatcaa 3780
gagcttgcga ggtcggatag agcttattgg cttgagcgct ttaagcatat gcctcctgag 3840
ttggaattgc cgatagattt ttcacgaggt tcgatgctta gtttcaaagg tgcatccatt 3900
cacagagatt ttgaattgcg ttgggatgct atttcgcata aggcagcgac ccttgataca 3960
acacctttta cactctttct tgcagcactt tatggggtgt tgcataccta ttcaagctca 4020
acagatattg taattggttc tcctgtctca gggcggattc atcctgatat tcaaaaaaca 4080
tgtggcattt tcataaacac gctcccactt agggtaacct ttagtcctac agaaaacttt 4140
ttaaccctag tagccagaac aaaaacagcc atcaccgaag ggcatgatca tcaactatac 4200
ccctttgata ggcttgttaa tgaacttgaa cttgctagaa gaacagacca tcagcctctt 4260
tttgatgtta tgctcttgct tcaagatacg gttgaagaga caatgcaagc acacgatttt 4320
acgcttaaag caagagggct tgagagctct aattcacatt ttgatatgac atttttttatc 4380
agtcaaatgg gtggtaatat tcgtcttgac attgagtaca acacctcttt atttacgaaa 4440
gaaagtgccc tacgttttgc tcggcactat gaaaattatc ttaaaaatgc gctgcgtcaa 4500
cccagcacta tgctcttgga tatacgttgt ataacccctg aagaaacgaa cttcttagcg 4560
caggtttcaa aagggttgcg cagaaatctt cctcaaaaaa gctttatcca ctgttttctt 4620
gaacaagccc agaagtttcc ttacaaagag gcagtggtaa cgcctttggg tcggtttagc 4680
tatgcagata ttgcgcaaaa agccattgcc atcgctgctg atattcaaag cgctggtatc 4740
gaaaaaggtg attttgtagg tcttacatgt aaaagggatg aaaacttagt agcaggaatg 4800

```
cttgggatta tgatgagtgg tgcaagttat gtttttatgt ctgctgaact gcctgaaaaa     4860
aggtttgcta aaattgtaaa aactgcccat attaaaaggg tatatgcatc cacggaagtt     4920
ccctttgtta ttcaaaatat cacacgtagg atcgatgatg tagcgcctgt ggcgtttgtg     4980
ccagattctt tttctacgat ggcatattgc atttttactt ctggcacaac aggggagcca     5040
aagggagtgc tcatcacaag ggaaaaccta gaaaaccttg tttttgcaac cgatatagaa     5100
atctacaaac aaagcgataa aactttacgt gaattgtgtg ctgtctcagg tgcttttgac     5160
gtttcgatta agcaagtttg tgcggccctt tcttgcggac acaccctttg tatgccagac     5220
gatacaacgc tgtatgaccc ttttttactt cttgagttca ttaaaaaaga gcagattcat     5280
ctgctggata tatcgccctc tcttttactg atgcttatgg aagtagggct ttgtgaaacg     5340
ccattaccct atgttaaaaa actgctcata ggctctgaag ctgttcagtt ttctgtgata     5400
aaacgattta tgcatgccca tccaaacgta gaggttttca attgttatgg tcctagtgaa     5460
tgcaccgttg agtctgtgag actaaagata gaggcaacac gagaataccc tcatattctc     5520
cccataggtc acccctgcct taacagcaat gcttatgtgc ttgatacgca tatgaacctt     5580
tgttgcgaag gtgtttatgg ggaaatccat cttggtgggg catgtgtggg ggcaggatac     5640
agtaacgaga caaaaaatcg gtttacatat tttgagaatg aacgtgtcta tgcaacaggc     5700
gatataggtc gctataaccg ttttggcgag atagaaattg cggggagaaa agatactcag     5760
cttaaaataa gagggtatcg tattgagcca gaggagatag aacattgcat tcttttagca     5820
ccaggtgttg ccttggctag tattgcagtt ttcaaacaag gcactttgga tgagatggca     5880
gccttttaca caggaacagc agacccagaa gaggttaaaa aacatgtcgc ttactttgtg     5940
ccttcttatg ccttgcctgc agtctttaag aaaatagata aaatgccact cagtgcaggt     6000
ggcaaaatag ataaaaaggc actcttagca gagcttgtct ttaacgaaaa acaaccagtt     6060
caacccgaag gacttcatct cgaaattgca tccttatggc acgaccttct gggtcattct     6120
ggttttggca tggaagatac attctttgat gctggaggaa attctgtttt gctggttcaa     6180
ctttacagta ggctgaataa ggcctatcca gatgttttta ccctagcagg tctttttttcc     6240
aaaagcagta ttgaagctca agccgaagcc attaaagtat attctatgac agatactttg     6300
atagagggaa tcacactccc ctctcatgcc cttagtcgtg aggcaaaagg caatacaagg     6360
ctttcatcgg cattacatgt aacgttgagt attcctcagg catcggctta tgcgatgtac     6420
cttatgttcg agctttatgg catagaagac ggggtgagtg ctgttattaa aaaaggtatt     6480
tgctatcttt gccgtgtcaa tcttcgtggg gttgaagata tagatgcgct tataaaaaga     6540
tgtgcggaca atttagagga ttcaaaaagc atacgaccac gagaaggatg tatcccctta     6600
gtgttatgtg atagtgaaat tttagcagag gagtacgtcg ctcaaaatgg tattgttttt     6660
gtgatgcaaa acggttttgt tgaggggtat ttttctgaaa aaatagacag caatgaagct     6720
```

gttaagataa tggagacgtt tatcatgatt atacaggcag cctcatgaaa aaaaaacttg 6780
accttaaaaa tgccataaag ccagatattg aaatcaaaag cgaagcttta aaaggagatg 6840
tggctgttgt gggtattggt atacgtgtag gagacatcgt ggggccagaa gatttttttg 6900
atgcgctgct gcgtcaaaga gaatttgtta aaaaaccttc atggcagagg atgaaagaca 6960
cagaagtgtt tgctgagaca tcttctcaag aatttgcaga gcttgcttac attgaagaac 7020
acgataggtt tgacaacaga tttttcaaaa ttccaccgca caaagctgag cttatggatc 7080
cacaagagaa gattctgctt cagtgtgctg ttgaagcggc agaagatgct ggcttagggg 7140
gtgatgctct tagtggaaca aaaaccgttg tgtttgctgg tggcatggga agctttttttg 7200
tgtatgagaa tgctcttaaa gacatgagcg atgtcaacac acttcttgaa ctcttaaccc 7260
cttccatgag tgccgctaga attgctcatt ttatgaatct taaaggtggc gcaacggttg 7320
ttgacacggc ttgttcctct tcccttgccg ctatttttta tgcttatcgt gctgttgctt 7380
cgggtgaata ttcggctgct tttgctgttg cttcaaagct cattcttaaa ccctttgcgt 7440
attcaggcgt agagatagcc tctaaagatg gacgtacaag agcattcgat aaagaggcta 7500
gcggaacggg aggcggagaa gctgcggcgg ctgttgtcct taaatctttg gagcgagcac 7560
tgagcgatgg ggatgacatt tatgccgtta tcaaagcagg atcattgaat cataatggtt 7620
tttctgtaaa cattacagcc cctgatgcaa aggcgcaaac agaactcatc acgtcagcat 7680
ggcgcaatgc gcagataaag cctgaggaaa ttaattttat agaagcccat ggaacagcaa 7740
caaaaatagg cgacgccata gagatagaag cattgaatgc agcctttaga agatacactg 7800
acaaaaaagc attttgtacg gttggctctg tcaaatcaaa cgttggtcat acggataatg 7860
ccgcaggaat cgtggggttt attaaagccg ttctttctgt taaaaataga atctatcctg 7920
cgactgttca ctttaatgag cctcatccag cagttgattt taatggctca cccgtctacg 7980
ctacaggggt aaatgttcca ctccaaagtc ccatcattac agcaggcgtt agtgcatttg 8040
ggcttagtgg aaccaatgtg catatggtgc ttcagaatgc accacaaaaa aaacaattac 8100
ccagaccgct tccccctat atttttgcgc tttcggcaaa aacagaaata tcccttgata 8160
tgcttatcca aaaatgggca aaatcaaagc ttaatgctta tcatcctgct gatatttcag 8220
caacgcttct tcatggaaga gggcactatg aagtgcgtgt ggcttttgtt tttaatacat 8280
ctgaagaact cttagaaaaa cttcaaaagc ttactactaa agagccgtgc gatgtttttc 8340
aaggggtaca cagtattgtc aaggatgtgc gcaatcttaa agaaggtgcg ctgagcgttg 8400
aaaaagccaa agaaataggg catgcaatga aaaacgcctt gaagcaaggt gattttgagt 8460
ccgcactcgc actgtatgta aaaggggcag aaccatcgtt tgatggtgtt tttaaagatg 8520
cttatacatc aaagttacac ttgcctgtct atgcttttga agctactcgt agatggtttt 8580

ctcatcaaaa ggaggtacgt tttagtcttt caggtacgga tagggttaca acaggtgttt 8640
ttacggtttt tagatcgttt atctctttta catcagattc actggtgggt gaacatagcg 8700
ttggtgataa atgcgttatg cctgctagtg ggattataga tcaaattttt gatgctgcag 8760
aaaagcacta tggtcatttg gagtatcgca ttgaagcatt aaagcttctt actttttttgg 8820
agatacccaa agagggtagc tccgaggtgg ttattcatgt ctcaacgacc caagacgctg 8880
aagagtgttc gcttatgcag aacatagggg gtagctggaa agtgtgtgct cagtggagga 8940
tgcataaggg aaaatctttg cctttaaaat ccaaagaaac agatattgaa aacagatacc 9000
ctgaaaaata tttctcaaaa gagagatttg caaaccaaat gggtggtgtt gttgtaagcg 9060
ataaatggaa ctgcattgag tatgcaggca aaaggggtga gtcagcagct tctattttac 9120
aagttcctag ggatgatagg agcttttcag gagccttttt attttacccg cctatggcag 9180
atgccgcact gaattttcat cctgaggcta aaaatggtat ccccgcatcg tttggtcgaa 9240
tagagttttt taagcctttg ggtacagcat gtctctcttt tgccaaactt aaagaaaaga 9300
gcgataagta tgtggtcttg agcattgtta ttacggataa caatggtgaa ccatgcgttg 9360
tgattgatga ttatacgcta agactttcta cgagtgaggg gtatctgcac aaaaaagtat 9420
ggaattcttt agggaagatt gctccaaaag tgttcgaaga tgtttttgta tttgaggaaa 9480
gtgatgctga aaagcccctc tttcaagaaa gactcaagca aatttattca gtagcatctg 9540
tacgcattgt atacaaagta aatactctca acaagcctga aaaaacattg cgtactcttt 9600
ttgacttttt gtgctctctt tcaaaaatgg aaggtaagat agagcttatt atctcagcag 9660
atgagaggac acacttgctt catggtgcta tggcggcgat gggtcgaacc tttgaatttg 9720
aacatccttg tgcaagattc cgatttgttg aaacgtatga agagagcatt aatcttttttg 9780
atgtgtttca ttttcaagga tttagattta gggtttctaa tgggactctc cagaggttgg 9840
agattgaaga gtgtggtata gggcaagggt tggagttaaa aaacggtggt caatatctta 9900
ttacaggggg acttggtgga ttaggtcttt ttactgcaga atatgtgtct aaaaaagttg 9960
attgtgcatt cgttttgatg tcccgtagtg gatttgcacc acaagatgag tggaattatc 10020
tctctgagtt aaaaggcata tctgcggata ttatagaaaa gatacaaaaa gtaaaaaaca 10080
accattgttc agtgacaatt tttaaaggag atgtctcttt ggagcgtgat cttgcacgcc 10140
ttaaagaaca atttggtcat tttgatggga tttttcatgc cgcaggttct gctacagaga 10200
ggttttttat caaaaatgat ttaaacgctt ttttaagaac agtgttacca aaggtcagag 10260
gaacacttaa cgtgcttgag atgtttcaag atactggttt ttttctgctc ttctcatcgc 10320
tttctggctt gacatcggca cctggtcaag caggctattg tgctgcaaat gcttttatgg 10380
attctttggc agaagatatg cctgaaaaaa ttctttccgt gggttggcca cgatggagtg 10440
aagttggcat ggcaattgga cttactagca agggggatga ttatgaagct ataaacttaa 10500

```
aagaagcatc taggatactg gatattcttc ctgcgggggg atatgttgct gttggcaaaa    10560
gtcatgcgcc actgagacct ttactagaag aagcctcagt gttaaaaagc tcactcgttg    10620
cttctgcaaa aacgaagatg agtaagagcg ttgaactaac aggtgcagaa tcatttacgc    10680
acatgcaaat agatattggt actatctggg cgggtgaact tggatatgaa acaattgata    10740
tttatgagga ttacgaagat ttaggtggag attcgattgc atctctttcc attcatgaaa    10800
aattagaaaa gagcttaggc ataagtcttt ctttttcgga gttgtttgaa aataaaacaa    10860
ttgccactat ggctcaatac cttgagcaaa aaattcacca aaagaataag ccaacaaagg    10920
aggataatat ccttgttttg aggcaaaaag gtgaacgaaa gattatctgt tttccaccag    10980
ggacagttat gggaaatgtt tacgctgggg tagcaacggc tttgagtggt tatgcggttt    11040
atgctttgaa ttatattcag tcgaagaatc cccagagttt gtttgcagat atgacagagt    11100
ccgttcagcc agaaggtgaa ctgattttac tagggtattc tgtggggggc aatttagcct    11160
atgaaacagc gaaggttctt gaaaaaagag gcagaaggat tagagcactt atctttttgg    11220
acaactttcg acgacttgag ctttttgaac aaagtgatga agcataccaa aggggagcaa    11280
gggagtattt gagcattctt gaaaaaacac aaggaagcga cttagataaa gaggagattc    11340
tagcaagaat tgaacactac gacagattca ttgattcaag acgtgaacaa gatcttatta    11400
gtgctccaat ttatcttata aaggctgaaa aaagtgctgc caatgcccca atgaaaatct    11460
cacaagatgc atggggtgag cttacaacgc attttgaaat ctttcaagga gatggaaaac    11520
acatggagat gctttttggt gaatacttcg aaaaaaatat tgagattata agaagtattt    11580
taaacacatt aaagtaattg ctatttatct tactataaat agcaattaca ctcttaaagg    11640
agacgaagat ggcaacagaa gtaaaagaag aaaaagtggt agaaggatcc gtttttgaaa    11700
cggtaagttt cgatgagaga aaacaaaaaa ctgacgagat tgtcaagcgt catgtgtacg    11760
gtgctatggg tgtagggctt gtacctattc ctctcataga ttttattggt gtgacaagtg    11820
ttcaagtgga tatgctctca acattggcgc aatattatgg tgtgacattc aaaaaagaga    11880
tggcaaaaaa tctgatagga tctcttgtcg gtggagctgt tcctgctgga ttgggaatgc    11940
ccattgctag tattcttaag ggcataccac ttgtaggtca aacggtgggc gctcttggaa    12000
tgtctatggt tgctggtgct tcaacgtatg ccgtaggtag agtgtttgtc caacattttg    12060
aatcaggtgg aacattttta agttttgatc ctggtgcggt aagagagtat tttaaagaag    12120
agtttaatat aggcaaaaca gtcgcaaaag aagcctctgc aaagaaataa cgatgagctt    12180
atttctttat atttttcttt gccttgcaat aggggtattt ggtctgaaca agaagttggg    12240
attttggggc tattttttg catcactttt gctgacacct cttcttggga ttatcttgct    12300
ccttgcatca ggagagaaga aaagcagaac cgattaagtc atgcaccttg aaactgtctt    12360
```

actacggtat tgcctttttg atgcgtatgg cgaagctaac ccgtgggtgt ctcttatgcc 12420

tgaatgcaac aggaaaagat gcacccactt taggaaggac gaatcaaaaa taaatcatgc 12480

agctgttagg ctcttacttg catgtgcatt gggcaaaaaa agagacatta ttgctaagat 12540

tagaaaaacg gatgcaggaa agccttttat agataaaatg ccttttttta gcttgagtca 12600

tacaacaaaa gctgtagcgg taagtacatg taaaaatcag cccacaggtg ttgatgcaga 12660

gttattaagg cacattgatg ttaatgattt tagccactat ttatcaaggg aagagaagga 12720

taagattaag ggttcatctg aggagttttt aaggctgtgg tgtacgaaag aagctgtttt 12780

taaagcagct ggaactggac tgcgtgaacc ttttacggat gtggttgtta tgccagattt 12840

tgctcttttt gatcataaca catggtacta taaaaccttg attgtggaga gtgtggtaat 12900

gagtgtttgt tgtgcaagac cttttaagat tgatatacaa gagcttaaac cgtgcgaatt 12960

acgggcttta gattaatgtg gatccgaatt cgagctccgt cgacaagctt gcggccgcac 13020

tcgagagcgc ttggagccac ccgcagttcg aaaaataatg agcttgacct gtgaagtgaa 13080

aaatggcgca cattgtgcga catttttttt gtctgccgtt taccgctact gcgtcacgga 13140

tctccacgcg ccctgtagcg gcgcattaag cgcggcgggt gtggtggtta cgcgcagcgt 13200

gaccgctaca cttgccagcg ccctagcgcc cgctcctttc gctttcttcc cttcctttct 13260

cgccacgttc gccggctttc cccgtcaagc tctaaatcgg gggctccctt tagggttccg 13320

atttagtgct ttacggcacc tcgaccccaa aaaacttgat tagggtgatg gttcacgtag 13380

tgggccatcg ccctgataga cggtttttcg ccctttgacg ttggagtcca cgttctttaa 13440

tagtggactc ttgttccaaa ctggaacaac actcaaccct atctcggtct attcttttga 13500

tttataaggg attttgccga tttcggccta ttggttaaaa aatgagctga tttaacaaaa 13560

atttaacgcg aattttaaca aaatattaac gcttacaatt tcaggtggca cttttcgggg 13620

aaatgtgcgc ggaaccccta tttgtttatt tttctaaata cattcaaata tgtatccgct 13680

catgagacaa taaccctgat aaatgcttca ataatattga aaaaggaaga gtatgagtat 13740

tcaacatttc cgtgtcgccc ttattccctt ttttgcggca ttttgccttc ctgtttttgc 13800

tcacccagaa acgctggtga aagtaaaaga tgctgaagat cagttgggtg cacgagtggg 13860

ttacatcgaa ctggatctca acagcggtaa gatccttgag agttttcgcc ccgaagaacg 13920

ttttccaatg atgagcactt ttaaagttct gctatgtggc gcggtattat cccgtattga 13980

cgccgggcaa gagcaactcg gtcgccgcat acactattct cagaatgact tggttgagta 14040

ctcaccagtc acagaaaagc atcttacgga tggcatgaca gtaagagaat tatgcagtgc 14100

tgccataacc atgagtgata acactgcggc caacttactt ctgacaacga tcggaggacc 14160

gaaggagcta accgcttttt tgcacaacat gggggatcat gtaactcgcc ttgatcgttg 14220

ggaaccggag ctgaatgaag ccataccaaa cgacgagcgt gacaccacga tgcctgtagc 14280

aatggcaaca acgttgcgca aactattaac tggcgaacta cttactctag cttcccggca 14340
acaattgata gactggatgg aggcggataa agttgcagga ccacttctgc gctcggccct 14400
tccggctggc tggtttattg ctgataaatc tggagccggt gagcgtggct ctcgcggtat 14460
cattgcagca ctggggccag atggtaagcc ctcccgtatc gtagttatct acacgacggg 14520
gagtcaggca actatggatg aacgaaatag acagatcgct gagataggtg cctcactgat 14580
taagcattgg taggaattaa tgatgtctcg tttagataaa agtaaagtga ttaacagcgc 14640
attagagctg cttaatgagg tcggaatcga aggtttaaca acccgtaaac tcgcccagaa 14700
gctaggtgta gagcagccta cattgtattg gcatgtaaaa aataagcggg ctttgctcga 14760
cgccttagcc attgagatgt tagataggca ccatactcac ttttgccctt tagaagggga 14820
aagctggcaa gattttttac gtaataacgc taaaagtttt agatgtgctt tactaagtca 14880
tcgcgatgga gcaaaagtac atttaggtac acggcctaca gaaaaacagt atgaaactct 14940
cgaaaatcaa ttagcctttt tatgccaaca aggtttttca ctagagaatg cattatatgc 15000
actcagcgca gtggggcatt ttactttagg ttgcgtattg gaagatcaag agcatcaagt 15060
cgctaaagaa gaaagggaaa cacctactac tgatagtatg ccgccattat tacgacaagc 15120
tatcgaatta tttgatcacc aaggtgcaga gccagccttc ttattcggcc ttgaattgat 15180
catatgcgga ttagaaaaac aacttaaatg tgaaagtggg tcttaaaagc agcataacct 15240
ttttccgtga tggtaacttc actagtttaa aaggatctag gtgaagatcc tttttgataa 15300
tctcatgacc aaaatccctt aacgtgagtt ttcgttccac tgagcgtcag accccgtaga 15360
aaagatcaaa ggatcttctt gagatccttt ttttctgcgc gtaatctgct gcttgcaaac 15420
aaaaaaacca ccgctaccag cggtggtttg tttgccggat caagagctac caactctttt 15480
tccgaaggta actggcttca gcagagcgca gataccaaat actgtccttc tagtgtagcc 15540
gtagttaggc caccacttca agaactctgt agcaccgcct acatacctcg ctctgctaat 15600
cctgttacca gtggctgctg ccagtggcga taagtcgtgt cttaccgggt tggactcaag 15660
acgatagtta ccggataagg cgcagcggtc gggctgaacg gggggttcgt gcacacagcc 15720
cagcttggag cgaacgacct acaccgaact gagataccta cagcgtgagc tatgagaaag 15780
cgccacgctt cccgaaggga gaaaggcgga caggtatccg gtaagcggca gggtcggaac 15840
aggagagcgc acgagggagc ttccaggggg aaacgcctgg tatctttata gtcctgtcgg 15900
gtttcgccac ctctgacttg agcgtcgatt tttgtgatgc tcgtcagggg ggcggagcct 15960
atggaaaaac gccagcaacg cggccttttt acggttcctg gccttttgct ggccttttgc 16020
tcacatgacc cgaca 16035